(19) Europäisches Patentamt
European Patent Office
Office européen des brevets

(11) Veröffentlichungsnummer: **0 362 520 B1**

(12) # EUROPÄISCHE PATENTSCHRIFT

(45) Veröffentlichungstag der Patentschrift: **26.10.94**

(21) Anmeldenummer: **89114990.8**

(22) Anmeldetag: **14.08.89**

(51) Int. Cl.5: **C07K 7/08**, C12P 21/04, A61K 37/02, C12N 1/20, //(C12N1/20,C12R1:07), (C12P21/04,C12R1:07)

(54) **Ein neues Antibiotikum, Mersacidin, ein Verfahren zur seiner Herstellung und seine Verwendung als Arzneimittel.**

(30) Priorität: **17.08.88 DE 3827868**

(43) Veröffentlichungstag der Anmeldung:
**11.04.90 Patentblatt 90/15**

(45) Bekanntmachung des Hinweises auf die Patenterteilung:
**26.10.94 Patentblatt 94/43**

(84) Benannte Vertragsstaaten:
**AT BE CH DE ES FR GB GR IT LI LU NL SE**

(56) Entgegenhaltungen:
**EP-A- 0 192 828**

(73) Patentinhaber: **HOECHST AKTIENGESELLSCHAFT**

**D-65926 Frankfurt (DE)**

(72) Erfinder: **Chatterjee, Sukumar, Dr.**
**G-32, Hoechst Ouarters**
**Darga Road**
**Mulund (West) Bombay 400 082 (IN)**

Erfinder: **Chatterjee, Sugata, Dr.**
**H1/2 Hoechst Ouarters**
**Darga Road**
**Mulund (West) Bombay 400 082 (IN)**
Erfinder: **Ganguli, Bimal Naresh, Dr.**
**"Saurayuth" 173 Central Avenue**
**Chembur Bombay 400 071 (IN)**
Erfinder: **Chatterjee, Deepak Kumar, Dr.**
**Sheetal Bungalow No. 2**
**Mahatma Phule Road**
**Mulund (East) Bombay 400 081 (IN)**
Erfinder: **Jani, Rajendra Kumar Hariprasad, Dr.**
**K-4 Hoechst Ouarters, Darga Road**
**Mulund (West)**
**Bombay, 400 082 (IN)**
Erfinder: **Rupp, Richard Helmut, Dr.**
**Roederweg 16a**
**D-6240 Königstein/Taunus (DE)**
Erfinder: **Fehlhaber, Hans-Wolfram, Dr.**
**Thomas-Mann-Strasse 5a**
**D-6270 Idstein (Taunus) (DE)**

EP 0 362 520 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Erfinder: **Kogler, Herbert, Dr.**
**Breslauer Strasse 39**
**D-6233 Kelkheim (Taunus) (DE)**
Erfinder: **Seibert, Gerhard, Prof Dr.**
**Gläserweg 21**
**D-6100 Darmstadt (DE)**
Erfinder: **Teetz, Volker, Dr.**
**An der Tann 20**
**D-6238 Hofheim am Taunus (DE)**

## Beschreibung

Die vorliegende Erfindung betrifft ein neues Antibiotikum, das als Mersacidin bezeichnet wird, ein Verfahren zu dessen Herstellung aus dem Eubakterium Bacillus species Y-85, 54728 (hinterlegt am 6.5.1988 gemäß den Bedingungen des Budapester Vertrags bei der Deutschen Sammlung für Mikroorganismen unter der Nummer DSM 4584), seinen Varianten und Mutanten, sowie die Verwendung von Mersacidin als Arzneimittel.

Das erfindungsgemäße Mersacidin ist ein cyclisches Polypeptid der Formel I.

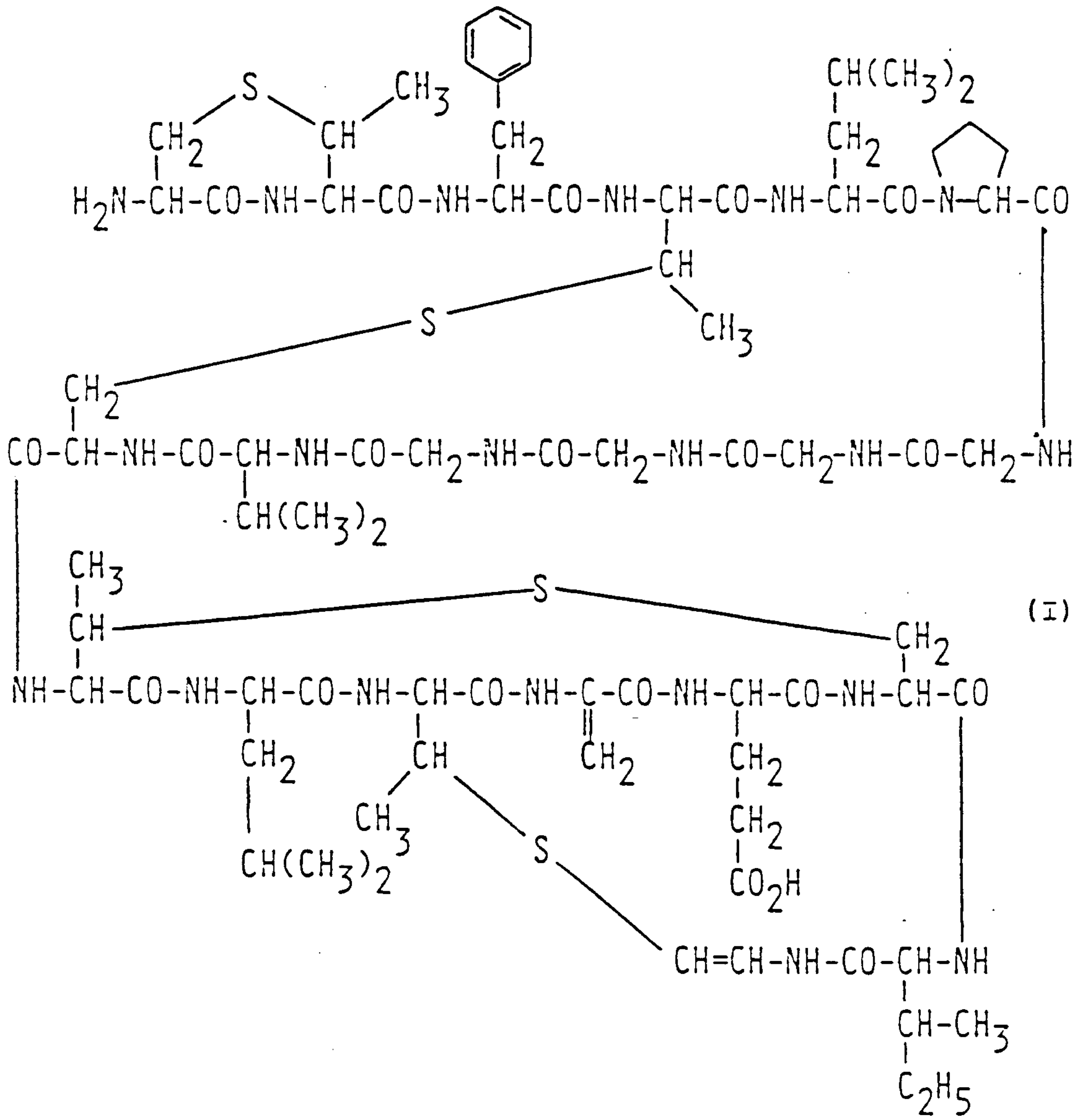

(I)

Die Konfiguration der chiralen C-Atome ist in Formel II verdeutlicht.

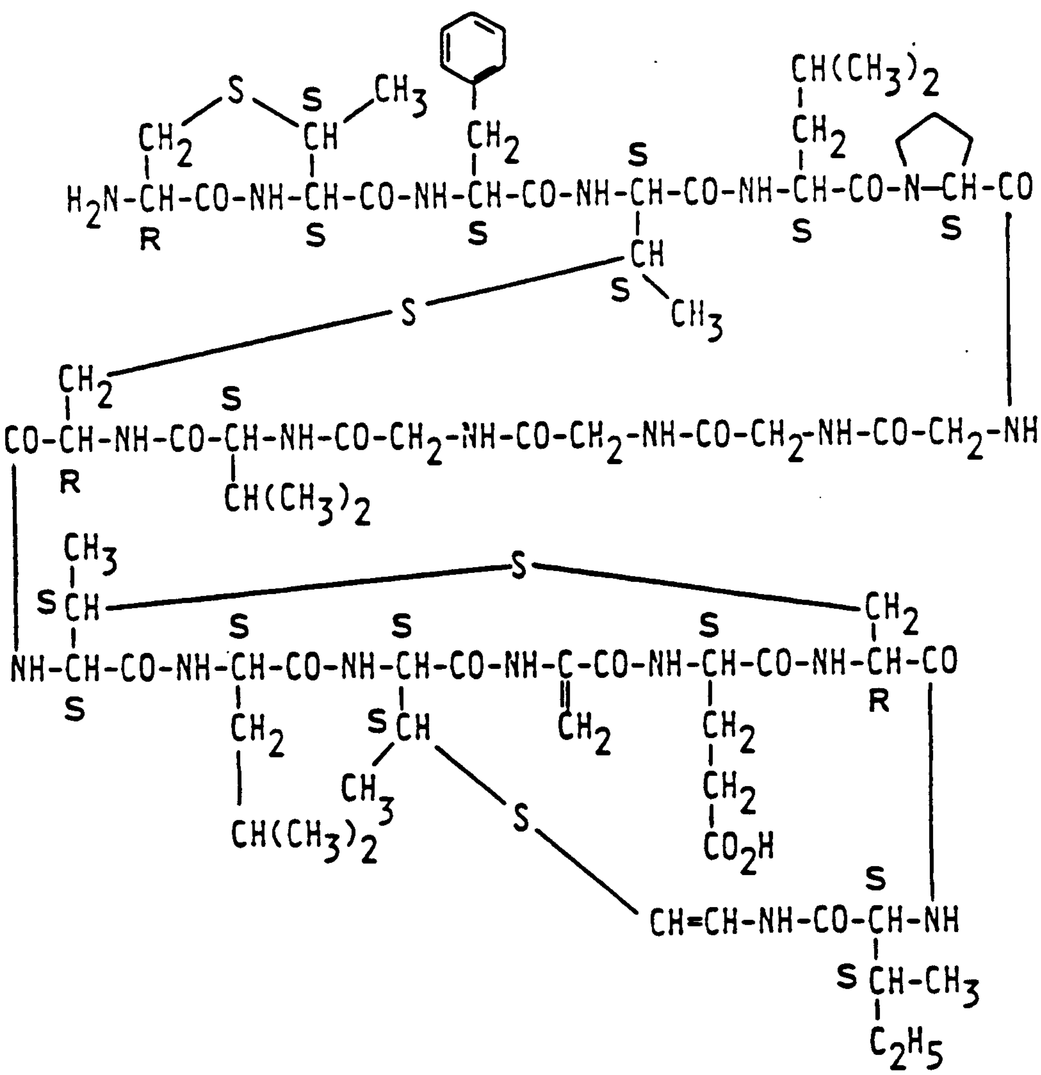

(II)

Die vorliegende Erfindung bezieht sich nicht nur auf das Mersacidin sondern auch auf dessen physiologisch verträgliche Salze und offensichtliche chemische Äquivalente.

Cyclische Peptidantibiotika werden im CRC Handbook of antibiotics (Antibiotika-Handbuch), Band IV, S. 263-424, Abschnitt "Cyclische Peptide" beschrieben. Aus dem Genus Bacillus isolierte Antibiotika werden auch in "Antibiotics, origin, nature and properties [Antibiotika, Ursprung, Charakter und Eigenschaften]", herausgegeben von Korzybski, T. et al., 1978, Band III, S. 1529-2078, beschrieben. Polypeptidantibiotika aus anderen mikrobiellen Quellen werden in der gleichen Literaturstelle, Band I, S. 311-491 beschrieben.

Allen verfügbaren Daten nach, unterscheidet sich Mersacidin jedoch eindeutig von allen in den obigen Literaturstellen beschriebenen cyclischen Polypeptidantibiotika. In den Chemical Abstracts ist keine Verbindung registriert, die das Molgewicht und die Aminosäurezusammensetzung aufweist wie Mersacidin.

Das für die Herstellung von Mersacidin verwendete Eubacterium, Kultur-Nummer Hoechst India Limited Y-85,54728, hiernach Y-85,54728 genannt, wurde aus einer in Mulund (Salzpfanne), Maharashtra, Indien, gewonnenen Bodenprobe isoliert und als Bacillus-Art identifiziert.

Physiologisch verträgliche Salze des Mersacidins lassen sich auf allgemein bekannte Weise mit verschiedensten Verbindungen bilden, z.B. mit organischen Aminen, wie z. B. Triethylamin oder Tri-(2-hydroxyethyl)-amin, mit Alkali- und Erdalkalimetallen, wie Na, K, Mg und Ca, mit anorganischen Säuren, wie z. B. Salzsäure, Schwefelsäure oder Phosphorsäure und mit organischen Säuren, wie z. B. Essigsäure, Zitronensäure, Benzoesäure, Maleinsäure, Fumarsäure, Weinsäure oder p-Toluolsulfonsäure.

Gegenstand der vorliegenden Erfindung ist weiterhin ein Verfahren zur Herstellung eines neuen Antibiotikums, Mersacidin, aus Kultur-Nummer Hoechst India Limited Y-85,54728 sowie seinen Mutanten

und Varianten.

Das besagte Verfahren umfasst die Kultivierung von Kultur-Nummer Y-85,54728, seiner Mutanten und/oder Varianten unter aeroben Bedingungen in einem Kohlenstoff- und Stickstoffquellen, anorganische Nährsalze und Spurenelemente enthaltenden Nährmedium sowie die Isolierung und Reinigung des besagten Antibiotikums aus der Kulturbrühe.

Bei den Kohlenstoffquellen kann es sich beispielsweise um Galaktose, Glycerin, Saccharose oder Glukose handeln. Galaktose wird als Kohlenstoffquelle bevorzugt. Als Stickstoffquellen werden Hefeextrakt, Rinderextrakt, Maisquellwasser, Casaminosäure oder anorganische Stoffe wie zum Beispiel Ammoniumsalze bevorzugt. Maisquellwasser wird als Stickstoffquelle besonders bevorzugt. Bei den anorganischen Nährsalzen kann es sich z. B. um Natriumhydrogenphosphat, Kaliumhydrogenphosphat, Calciumchlorid oder Magnesiumsulfat handeln. Spurenelemente können z. B. Eisen-, Mangan-, Kupfer-, oder Zinksalze oder andere Metallsalze sein.

Die Kultivierung von Kultur-Nummer Y-85,54728 wird vorzugsweise bei Temperaturen zwischen 26-29 °C und pH-Werten zwischen ca. 6,5 und 7,2 durchgeführt. Kultur-Nummer Y-85,54728 wird besonders bevorzugt bei 28 °C (± 1 °C) und pH 7,2 kultiviert.

Die Kultivierung wird vorzugsweise ca. 60 bis 72 Stunden lang durchgeführt, wobei das erfindungsgemässe Antibiotikum in optimaler Ausbeute anfällt. Die Kultivierung wird besonders bevorzugt 66 Stunden lang unter Submersbedingungen in Schüttelkolben sowie in Laborfermentern durchgeführt. Bei Bedarf kann ein Schaumdämpfer in den Fermentern verwendet werden (z. B. Desmophen®, Polyole der Fa. Bayer AG, Leverkusen). Der Ablauf der Kultivierung und die Bildung des erfindungsgemässen Mersacidins ist durch Messung der Bioaktivität der Kulturbrühe gegen Staphylococcus aureus 209 P, Staphylococcus aureus R 85 und Alcaligenes faecalis mit der bekannten Agarplattendiffusionsnachweismethode feststellbar (vgl. z. B. Oxoid-Handbuch 1972, 2. Auflage, herausgeber Oxoid Limited, London, England).

In der resultierenden Kulturbrühe findet sich Mersacidin nur im Kulturfiltrat, das durch Zentrifugieren von der Zellmasse abgetrennt wird. Mersacidin lässt sich mit einem oder mehreren bekannten Verfahren, wie beispielsweise hydrophobee Wechselwirkungs-Chromatographie z.B. auf polymeren Adsorptionsmitteln wie ®Diaion HP-20 (Mitsubishi Chemical Industries, Japan) oder ®Amberlite XAD-2(R), XAD-7(R) (Polymeres Adsorptionsmittel aus einer Matrix aus Polystyrol, Acrylester oder Aminoxid mit einem durchschnittlichen Porendurchmesser von (40-225)$\cdot 10^{-10}$m, Rohm & Haas Co., USA), oder Extraktion mit mit Wasser nicht mischbaren Lösungsmitteln wie Äthylacetat oder Butanol aus dem Kulturfiltrat isolieren. Das bevorzugte Verfahren ist Adsorption an Diaion HP-20 mit nachfolgender Desorption der Verbindung mit geeigneten organischen Lösungsmitteln wie z.B. Methanol, Acetonitril oder wässrigen Kombinationen dieser Lösungsmittel. Methanol wird als Lösungsmittel zur Elution von Mersacidin bevorzugt.

Die Entfernung des Lösungsmittels aus den aktiven Eluaten ergibt rohes Mersacidin. Die weitere Reinigung läßt sich durch Chromatographie an Substanzen wie Kieselgel, modifiziertes Kieselgel, Cellulose oder ®Sephadex LH-20 (lipophiles Gelfiltrationsmaterial, Hersteller Pharmacia Fine Chemicals AB, Schweden) erreichen. Chromatographie des rohen Mersacidins auf Kieselgel unter Verwendung von Acetonitril-Wasser-Gemischen zur Elution durch stufenweise Erhöhung der Wasserkonzentration um jeweils 5 bis 10%, stellt das bevorzugte Verfahren dar. Die aktiven Eluate (mit dem Bionachweisverfahren ermittelt) werden zur halbreinen Verbindung eingeengt. Das so erhaltene halbreine Antibiotikum kann z.B. durch Chromatographie an lipophilem Gelfiltrationsmaterial, wie z.B. Sephadex® LH-20 unter Verwendung von Lösungsmitteln wie z.B. Wasser, MeOH, $CHCl_3$, Hexan oder deren entsprechenden Kombinationen, gegebenenfalls mit nachfolgender Behandlung mit Aktivkohlepulver, oder durch präparative Hochdruckflüssigkeitschromatographie auf einer Kieselgelsäule unter Verwendung von Lösungsmitteln wie z.B. MeOH, $CH_3CN$, Wasser oder deren entsprechende Kombinationen weiter gereinigt werden. Präparative Hochdruckflüssigkeitschromatographie auf einer Säule mit HPLC-Material mit Octadecyltrichiorosilan-Gruppen (wie z.B. das Produkt Hypersil® der Fa. Shandon, USA) unter Verwendung eines Gemisches (70:30) aus $CH_3CN$ und Wasser ist das bevorzugte Verfahren. Nach Entfernung des organischen Lösungsmittels aus den aktiven Eluaten z.B. durch Abdestillieren im Vakuum und nachfolgende Lyophilisierung zur Entfernung des Wassers erhält man Mersacidin als weisses Pulver.

Mersacidin ist durch seine antibakterielle Wirksamkeit (vgl. Wirksamkeitstest) für den Einsatz als Arzneimittel qualifiziert. Erfindungsgegenstand sind demzufolge auch Arzneimittel mit einem Gehalt an Mersacidin neben üblichen, allgemein bekannten Hilfs- und/oder Trägerstoffen, sowie die Verwendung von Mersacidin zur Herstellung von Arzneimitteln mit antibiotischer und/oder immunsuppressiver Wirkung in an sich bekannter Weise.

Die nachfolgenden Beispiele dienen zur Erläuterung der vorliegenden Erfindung.

**BEISPIEL 1**

Isolierung von Kultur Y-85,54728 aus dem Boden.

(a) Zusammensetzung des Isolationsnährmediums

| | |
|---|---|
| Proteose Pepton | 20,0 g |
| $K_2SO_4$ | 1,5 g |
| $MgSO_4.7H_2O$ | 1,5 g |
| Glycerin | 10,0 g |
| Agarpulver | 15,0 g |
| Entmineralisiertes Wasser | 1 Liter |
| pH-Wert | 6,8 |

(b) Boden-Ausstrich und Isolierung

10 g einer in einer Salzpfanne in Mulund gewonnenen Bodenprobe wurden 90 ml sterilisiertes entmineralisiertes Wasser in einem 250 ml-Erlenmeyerkolben hinzugefügt, und der Kolben wurde 2 Stunden lang auf einer Rundschüttelmaschine bei 220 Umdrehungen geschüttelt. Die obige Bodensuspension wurde dann in Stufen von 10 bis $10^{-5}$ reihenverdünnt. 1 ml Suspension der letzten Verdünnung wurde in die Mitte einer sterilen Petri-Glasplatte (6 Zoll Durchmesser) gegeben, wonach ungefähr 50 ml des obigen, auf 45 °C abgekühlten Isoliermediums zugegossen und die Platte kräftig geschüttelt wurde. Das Gemisch aus Bodensuspension und Medium ließ man sich absetzen, und es wurde 3 Tage lang bei 28 °C (± 1 °C) inkubiert. Die Petriplatte wurde in regelmässigen Abständen begutachtet und Kultur-Nummer Y-85,54728 aus den wachsenden Mikroorganismen isoliert.

**BEISPIEL 2**

Erhaltung der Kultur Y-85,54728

Zusammensetzung des Erhaltungsmediums

Kultur-Nummer Y-85,54728 wurde auf Nähragarmedium der folgenden Zusammensetzung erhalten.

| | |
|---|---|
| Pepton | 10 g |
| Rinderextrakt | 3 g |
| Hefeextrakt | 3 g |
| Agarpulver | 15 g |
| Entmineralisiertes Wasser | 1 Liter |
| pH-Wert | 7,2 |

Nach gründlicher Auflösung der Inhaltsstoffe durch Erhitzen wurden diese in Reagenzgläser Verteilt und dann bei 121 °C zwanzig Minuten lang sterilisiert. Die Reagenzgläser wurden abgekühlt und in Schräglage erstarren gelassen. Die Schrägagarkulturen wurden mit Hilfe einer Drahtschleife mit der Kultur-Nummer Y-85,54728 versetzt und bei 28 °C (± 1 °C) bis zur Sichtbarkeit eines guten Wachstums inkubiert. Die gut gewachsenen Kulturen wurden bei +8 °C im Kühlschrank aufbewahrt.

**BEISPIEL 3**

Fermentation von Kultur Y-85,54728 in Schüttelkolben.

Zusammensetzung des Saatkulturmediums

| | |
|---|---|
| Casaminosäure | 5 g |
| Maisquellwasser | 5 g |
| Glycerin | 20 g |
| Galaktose | 10 g |
| Entmineralisiertes Wasser | 1 Liter |
| pH-Wert | 7,2 |

Jeweils 25 ml des obigen Saatkulturmediums wurden in 250 ml-Erlenmeyerkolben verteilt und bei 121 °C 20 Minuten lang im Autoklaven erhitzt. Die Kolben wurden abgekühlt und jeweils mit einer Schleife der obengenannten gut gewachsenen Kultur aus Beispiel 2 beimpft und bei 28 °C (± 1 °C) 24 Stundenlang mit 220 Umdrehungen pro Minute geschüttelt. Die erhaltene Kulturlösung wurde als Saatkultur zur Beimpfung der Herstellungskolben wie nachstehend beschrieben verwendet.

Herstellung des Antibiotikums Mersacidin in Schüttelkolben

Das Herstellungsmedium entsprach dem in Beispiel 3 beschriebenen Saatmedium. Jeweils 100 ml des Mediums wurden in 500 ml-Erlenmeyerkolben verteilt und bei 121 °C 20 Minuten lang im Autoklaven erhitzt. Die Kolben wurden abgekühlt, danach mit der obengenannten Saatkultur (1% V/V) beimpft. Die Fermentation wurde in einer Rundschüttelmaschine bei 220 Umdrehungen pro Minute und einer Temperatur von 28 °C (± 1 °C) 66 Stunden lang durchgeführt.

Die Produktion des Antibiotikums wurde durch das gegen Staphylococcus aureus 209 P, Staphylococcus aureus R 85 und Alcaligenes faecalis unter Verwendung des Plattendiffusionsverfahrens in bekannter Weise geprüfte Bioaktivitätsprofil überwacht. Nach der Aberntung wurde die Kulturbrühe zentrifugiert und Mersacidin aus dem Kulturfiltrat isoliert und wie nachfolgend beschrieben gereinigt.

**Beispiel 4**

Kultivierung von Kultur-Nummer Y-85,54728 in Fermentern

Stufe 1 Herstellung der Saatkultur in Schüttelkolben

Das Saatkulturmedium aus Beispiel 3 (100 ml) wurde in 500 ml-Erlenmeyerkolben mit einem auf 6,8 eingestellten Vorsterilisations-pH-Wert vorgelegt. Letzteres wurde bei 121 °C 20 Minuten lang in einem Autoklaven sterilisiert, abgekühlt und mit einer Schleife der gut gewachsenen Kultur aus Beispiel 2 beimpft. Die Kolben wurden bei 28 °C (± 1 °C) 24 Stunden lang in einer Rundschüttelmachine bei 220 Umdrehungen pro Minute inkubiert. Diese Wachstumskultur wurde zur Beimpfung von kleinen Fermentern wie unten beschrieben verwendet.

Stufe 2 Herstellung der Saatkultur in kleinen Fermentern

10 Liter des Saatmediums (wie in Beispiel 3 beschrieben) mit einem auf 6,8 eingestellten Vorsterilisations-pH-Wert, wurden mit 0,04% (V/V) Desmophen als Schaumdämpfer in einem 15 Liter fassenden Edelstahlfermenter vorgelegt, bei 121 °C 36 Minuten lang in einem Autoklaven sterilisiert, abgekühlt und mit 4% (V/V) Saatgut aus der obigen Stufe 1 des Beispiels 4 unter aseptischen Bedingungen beimpft. Anschließend wurde 24 Stunden lang unter den folgenden Bedingungen kultiviert:

| | |
|---|---|
| Temperatur<br>Rührgeschwindigkeit<br>Belüftung | 28 °C (± 1 °C)<br>150 UpM<br>6 Liter pro Minute |

Die nach 24 Stunden gewachsene Kultur wurde zur Beimpfung des Herstellungsmediums aus Stufe 3 verwendet.

Stufe 3 Fermentation im Produktionsmassstab

100 Liter des Herstellungsmediums (entsprechend dem in Beispiel 3 erwähnten Saatmedium) mit auf 6,8 eingestelltem Vorsterilisierungs-pH-Wert und Zusatz von 0,06% Desmophen in einem 150 Liter fassenden Fermenter, oder 250 Liter Medium mit 0,06% Desmophen in einem 390 Liter fassenden Fermenter, wurden 28 Minuten lang bei 121 °C in situ sterilisiert und mit 4% der Saatkultur aus Stufe 2 beimpft.

Unter den folgenden Bedingungen wurde kultiviert:

| | |
|---|---|
| Temperatur:<br>Rührgeschwindigkeit: | 28 °C (± 1 °C)<br>80 bis 100 UpM |
| Belüftung: | 50 Liter pro Minute (bei 100 Liter-Fermenter)<br>125 Liter pro Minute (bei 390 Liter-Fermenter) |
| Erntezeit: | nach 66 Stunden |

Die Produktion des Antibiotikums wurde mit dem gegen Staphylococcus aureus 209 P, Staphylococcus aureus R 85 und Alcaligenes faecalis geprüften Aktivitätsprofil überwacht. Die Kulturbrühe wurde nach der Ernte zentrifugiert und das Antibiotikum wurde aus dem Kulturfiltrat wie nachfolgend beschrieben, isoliert und gereinigt.

**BEISPIEL 5**

Isolierung und Reinigung von Mersacidin

Ungefähr 100 Liter der geernteten Brühe wurden durch Zentrifugieren vom Myzel getrennt. Das erhaltene Filtrat (pH 6,8-7,0) wurde durch eine 5 Liter-Diaion HP-20-Säule geführt. Die Säule wurde zunächst mit 50 Litern entmineralisiertem Wasser gewaschen. Sie wurde danach nacheinander mit 70% $H_20$ in MeOH (40 Liter), 50% $H_20$ in MeOH (50 Liter) und 30% $H_20$ in MeOH (50 Liter) gewaschen und das Waschwasser jeweils verworfen. Die Säule wurde schliesslich mit 10 Litern MeOH eluiert. Die aktiven Eluate wurden unter vermindertem Druck zu einem braunen öligen Stoff eingeengt. Dieser wurde mit einem Liter Petroläther (Kochpunkt 40 bis 60 °C) verrieben, filtriert und das Filtrat verworfen. Dieser Vorgang wurde wiederholt, bis ein Pulver als Filterrückstand übrig blieb. Rohes Mersacidin wurde somit als bräunlich gelbes Pulver erhalten (12 g).

Rohes Mersacidin wurde dann einer Mitteldruck-Flüssigkeitschromatographie über 300 g Kieselgel (200 bis 300 mesh) in einer 5,5 x 53 cm großen Glassäule unterworfen. Die Säule wurde mit 1,5 Litern $CH_3CN$ gewaschen und danach mit 10% wässrigem $CH_3CN$ (3 Liter) und 15% wässrigem $CH_3CN$ (3 Liter) bei einer Strömungsgeschwindigkeit von 16 ml pro Minute eluiert. Es wurden 250 ml-Fraktionen gesammelt und durch UV-Nachweis bei einer Wellenlänge von 210 nm und durch mikrobielle Nachweisverfahren geprüft. 6 Fraktionen (1,5 Liter) der 15%-igen wässrigen $CH_3CN$-Eluate waren aktiv und wurden im Vakuum bei 35 °C zur Entfernung von $CH_3CN$ eingeengt und danach gefriergetrocknet, wobei 2 g halbreiner Stoff als gelbliches Pulver erhalten wurden.

Das so erhaltene halbreine Material wurde wiederum einer Mitteldruck-Flüssigkeitschromatographie auf Kieselgel (200 bis 300 mesh) in einer 4 x 54 cm grossen Glassäule unterworfen. Die Säule wurde nacheinander mit $CH_3CN$ (1 Liter), 5% wässrigem $CH_3CN$ (1 Liter) und 10% wässrigem $CH_3CN$ (4,5 Liter) bei einer Strömungsgeschwindigkeit von 30 ml pro Minute eluiert. 250 ml-Fraktionen wurden gesammelt und durch UV-Nachweis bei 210 nm und durch mikrobiellen Nachweis erfaßt. Mersacidin wurde durch Elution mit 10%-igem wässrigem $CH_3CN$ (750 ml) gewonnen, das dann unter vermindertem Druck bei 35 °C mit nachfolgender Gefriertrocknung eingeengt wurde, wobei 593 mg Mersacidin als weisses bis weissliches Pulver erhalten wurden.

Die Endreinigung des Mersacidin erfolgte durch Hochdruckflüssigkeitschromatographie auf einer 4 x 250 mm grossen Hypersil (10 µ)-Säule unter Verwendung von 30%-igem wässrigem $CH_3CN$ als Elutionsmittel und einer Strömungsgeschwindigkeit von 1,0 ml pro Minute. Die Fraktionen wurden mit einem UV-Nachweissatz bei 210 nm, erfasst. Unter diesen Bedingungen hatte Mersacidin eine Retentionszeit von ca. 3,5 Minuten. Die entsprechenden Fraktionen wurden gesammelt, im Vakuum bei 35 °C eingeengt und schließlich lyophilisiert, wobei 275 mg des Antibiotikums als weisses Pulver erhalten wurden. Die Reinheit von Mersacidin wurde auf einer 4 x 120 mm großen APS-Hypersil (5 µ)-Säule überprüft; Gemisch (70:30) aus $CH_3CN$ und $H_20$ als Elutionsmittel; Strömungsgeschwindigkeit1 ml pro Minute; Papiervorschubgeschwindigkeit 10 mm pro Minute; Nachweis bei 210 nm. Das Hochdruckflüssigkeitschromatogramm zeigt Figur 1.

Physikalisch-chemische Eigenschaften von Mersacidin

Mersacidin ist eine neue Verbindung deren Struktur durch ein Nonadecapeptid mit einem C-terminalen Vinylamid-Rest und vier intramolekulare Sulfidbrücken gekennzeichnet ist.

Tabelle 1 faßt die physikalisch-chemischen Eigenschaften von Mersacidin zusammen.

Tabelle 1

| Physikalisch-chemische Eigenschaften von Mersacidin | |
|---|---|
| Aussehen: | Weisses amorphes Pulver |
| Charakter: | Cyclisches Polypeptid |
| Schmelzpunkt: | ca. 240 °C (Zerfall) |
| $[\alpha]_D 20°$ | - 9,4° (c = 0,3, MeOH) |
| Summenformel: | $C_{80} H_{120} N_{20} O_{21} S_4$ |

- bestätigt durch hochauflösende Massenspektrometrie (FAB-Ionisierung, Matrix 3-Nitrobenzylalkohol)
- gemessenes m/z 1825,785 des $M + H^+$-Ions
- berechnetes m/z 1825,790 für
  $^{12}C_{80}\ ^{1}H_{121}\ ^{14}N_{20}\ ^{16}O_{21}\ ^{32}S_4$

Massenspektrum (FAB, Matrix 3-Nitrobenzylalkohol) Figur 2

$^1$H-NMR-Spektrum (270 MHz, $CD_3OD$): Fig. 3

$^{13}$C-NMR-Spektrum (100 MHz, $CD_3OH$): Tab. 2

**Tabelle 2 $^{13}$C-NMR Daten von Mersacidin (7 %ige Lösung in $CD_3OH$, 310 K)**

| Chemische Verschiebung (ppm) | Multiplizität | Strukturzuordnung |
|---|---|---|
| 10.42 | q | C-5 Ile (19) |
| 15.97 | q | C-4' Ile (19) |
| 18.51 | q | C-4 3-thio-Abu (2) |
| 18.68 | q | C-4' Val (11) |

**Fortsetzung Tabelle 2**

| Chemische Verschiebung (ppm) | Multiplizität | Strukturzuordnung |
|---|---|---|
| 19.77 | q | C-4 Val (11) |
| 20.13 | q | C-4 3-thio-Abu (4+15) |
| 21.28 | q | C-4 3-thio-Abu (13) |
| 22.43 | t | C-3 Cys (1) |
| 22.43 | q | C-5' Leu (5) |
| 23.17 | q | C-5' Leu (14) |
| 23.53 | q | C-5 Leu (5) |
| 23.58 | q | C-5 Leu (14) |
| 25.47 | d | C-4 Leu (5) |
| 25.78 | d | C-4 Leu (14) |
| 26.00 | t | C-4 Pro (6) |
| 26.68 | t | C-4 Ile (19) |
| 28.17 | t | C-3 Glu (18) |
| 30.85 | t | C-3 Pro (6) |
| 32.54 | d | C-3 Val (11) |
| 33.58 | t | C-3 Cys (12) |
| 35.21 | d | C-3 Ile (19) |
| 35.59 | t | C-4 Glu (17) |
| 37.14 | t | C-3 Cys (18) |
| 37.34 | t | C-3 Phe (3) |
| 39.39 | d | C-3 3-thio-Abu (2) |
| 40.93 | t | C-3 Leu (14) |
| 42.02 | t | C-3 Leu (5) |
| 42.81 | t | C-2 Gly (10) |
| 43.38 | t | C-2 Gly (9) |
| 43.59 | d | C-3 3-thio-Abu (4) |
| 44.10 | t | C-2 Gly (7) |
| 44.62 | t | C-2 Gly (8) |
| 45.50 | d | C-3 3-thio-Abu (13) |
| 48.67 | t | C-5 Pro (6) |
| 49.46 | d | C-3 3-thio-Abu (15) |
| 50.42 | d | C-2 Leu (14) |
| 52.71 | d | C-2 Leu (5) |
| 55.25 | d | C-2 Cys (18) |
| 55.72 | d | C-2 Cys (12) |

**Fortsetzung Tabelle 2**

| Chemische Verschiebung (ppm) | Multiplizität | Strukturzuordnung |
|---|---|---|
| 56.72 | d | C-2 Glu (17) |
| 57.42 | d | C-2 3-thio-Abu (15) |
| 57.81 | d | C-2 Phe (3) |
| 58.12 | d | C-2 Cys (1) |
| 59.07 | d | C-2 3-thio-Abu (2) |
| 59.08 | d | C-2 3-thio-Abu (4) |
| 59.19 | d | C-2 3-thio-Abu (13) |
| 61.60 | d | C-2 Pro (6) |
| 62.64 | d | C-2 Ile (19) |
| 63.14 | d | C-2 Val (11) |
| 103.84 | d | C-2 Thiovinylamid (20) |
| 112.41 | t | C-2 dehydro-Ala (16) |
| 127.97 | d | C-7 Phe (3) |
| 129.56 | d | C-1 Thiovinylamid (20) |
| 129.71 | d | C-6 Phe (3) |
| 130.22 | d | C-5 Phe (3) |
| 136.96 | s | C-4 Phe (3) |
| 138.15 | s | C-2 dehydro-Ala (16) |
| 166.56 | s | C-1 dehydro-Ala (16) |
| 171.16 | s | C-1 Ile (19) |
| 171.38 | s | C-1 Gly (10) |
| 171.44 | s | C-1 Cys (18) |
| 171.62 | s | C-1 3-thio-Abu (4) |
| 171.76 | s | C-1 3-thio-Abu (15) |
| 171.82 | s | C-1 Gly (9) |
| 171.84 | s | C-1 Leu (5) |
| 171.86 | s | C-1 3-thio-Abu (13) |
| 171.87 | s | C-1 3-thio-Abu (2) |
| 172.59 | s | C-1 Gly (8) |
| 172.74 | s | C-1 Leu (14) |
| 173.06 | s | C-1 Gly (7) |
| 173.76 | s | C-1 Cys (12) |
| 174.00 | s | C-1 Val (11) |
| 174.25 | s | C-1 Glu (17) |

**Fortsetzung Tabelle 2**

| Chemische Verschiebung (ppm) | Multiplizität | Strukturzuordnung |
|---|---|---|
| 174.61 | s | C-1 Cys (1) |
| 174.81 | s | C-1 Phe (3) |
| 175.62 | s | C-1 Pro (6) |
| 181.31 | s | C-5 Glu (17) |

Wirksamkeitstests:

a) Wirksamkeit von Mersacidin in vitro

Die biologischen Eigenschaften von Mersacidin als zur Hemmung des Wachstums von verschiedenen Mikroorganismen benötigte MHK-Werte sind in Tabelle 3 aufgeführt.

**Tabelle 3**

| Nummer | Testorganismus | MHK-Werte (µg/ml) |
|---|---|---|
| 1. | Staph. aureus 209P | 0,78 - 1,56 |
| 2. | Staph. aureus E 88 | 6,25 - 12,50 |
| 3. | Staph. aureus 3066 | 12,50 - 25,00 |
| 4. | Staph. aureus 20240 | 3,12 - 6,25 |
| 5. | Staph. aureus 20424 | 1,56 - 3,12 |
| 6. | Staph. aureus 503 | 0,78 - 1,56 |
| 7. | Staph. aureus 789 | 0,78 - 1,56 |
| 8. | Staph. aureus 722 | 3,12 - 6,25 |
| 9. | Staph. aureus SG 511 | 0,39 - 0,78 |
| 10. | Staph. aureus 20666 | 3,12 - 6,25 |
| 11. | Staph. aureus E 712 | 3,12 - 6,25 |
| 12. | Staph. aureus 285 | 0,78 - 1,56 |
| 13. | Staph. aureus R 85 | 0,39 - 0,78 |
| 14. | Staph. aureus 710 | 0,39 - 0,78 |
| 15. | Micrococcus luteus | 0,0975-0,195 |

**Fortsetzung Tabelle 3**

| Nummer | Testorganismus | MHK-Werte (µg/ml) |
|---|---|---|
| 16. | Bacillus subtilis | 0,39 - 0,78 |
| 17. | Streptococcus faecalis | 0,0975-0,195 |
| 18. | Str. D 21777 | 1,56 - 3,12 |
| 19. | S. epidermidis MRSE | 0,5 - 16,0 |
| 20. | S. epidermidis MSSE | 0,5 - 16,0 |
| 21. | Gruppe A Streptococci | 0,5 - 8,0 |
| 22. | Gruppe B Streptococci | 1,0 - 8,0 |
| 23. | Gruppe C Streptococci | 2,0 - 64 |
| 24. | Gruppe G Streptococci | 2,0 - 8,0 |
| 25. | Streptococci bovis | 4,0 - 32,0 |
| 26. | Streptococcus viridans | 0,5 - 32,0 |
| 27. | Streptococcus faecalis | 64,0 |
| 28. | Streptococcus pneumoniae | 1,0 - 4,0 |
| 29. | Corynebacterium JK | 2,0 - 4,0 |
| 30. | Listeria monocytogenes | 16,0 - 64,0 |
| 31. | Clostridium species | 0,5 - 16,0 |
| 32. | Peptostreptococci species | 0,5 - 8,0 |
| 33. | Propionobacterium genes | 1,0 - 16,0 |

b) Wirksamkeit von Mersacidin in vivo

Im in vivo-System von Labormäusen zeigte Mersacidin gute Aktivität. Bei subkutaner Verabreichung in einer Dosis von 9,37 und 25.00 mg/kg an experimentell mit Staphylococcus aureus SG 511 und S. aureus 710 (resistent gegen Methicillin) infizierten Labormäusen wurde eine 100%-ige Heilungsrate erzielt. Darüber hinaus wurde die akute Toxizität des Antibiotikums in Labormäusen geprüft. Selbst bei einer Dosis von 500 mg/kg (subkutan), die bisher höchste Prüfkonzentration, wurde keine Mortalität der Tiere beobachtet. Die Ergebnisse sind in Tabelle 4 aufgeführt.

Tabelle 4: In vivo-Wirksamkeit von Mersacidin gegen S. aureus SG 511-Infektion in Mäusen im Vergleich zu Vancomycin.

Verwendetes Impfmedium: 1,75 x $10^9$ g KBE/Maus

| Verbindung | Dosis/Anwendung mg/kg x 3, s.c. | Anzahl Mäuse geheilt/behandelt (prozentuale Heilungsrate) | Ungefähre $ED_{50}$, $ED_{90}$ mg/kg x 3 |
|---|---|---|---|
| Mersacidin | 9,37 | 53/53 (100) | |
| | 6,25 | 38/40 ( 95) | 2,59 |
| | 3,12 | 18/27 ( 67) | 5,38 |
| | 1,60 | 3/24 ( 12) | |
| | 0,80 | 0/27 ( 0) | |
| Vancomycin | 18,75 | 71/71 (100) | |
| | 12,5 | 59/61 ( 97) | 7,20 |
| | 9,37 | 48/53 ( 85) | 9,37 |
| | 6,25 | 13/44 ( 29) | |
| | 3,12 | 2/41 ( 5) | |

**In vivo-Wirksamkeit von Mersacidin gegen S. aureus E 710 (MRSA) in Mäusen, im Vergleich zu Vancomycin**

**Verwendetes Impfmedium: 5 x $10^9$ g KBE/Maus (1 MLD)**

| Verbindung | Dosis/Anwendung mg/kg x 3, s.c. | Anzahl Mäuse geheilt/behandelt (prozentuale Heilungsrate) | Ungefähre $ED_{50}$, $ED_{90}$ mg/kg x 3 |
|---|---|---|---|
| | 25,00 | 12/12 (100) | 10,81 |
| Mersadicin | 12,50 | 10/16 ( 63) | 19,59 |

| | | | |
|---|---|---|---|
| | 9,37 | 4/10 ( 40) | |
| | 6,25 | 1/10 ( 10) | |
| Vancomycin | 37,00 | 6/6 (100) | 18,94 |
| | 25,00 | 14/21 ( 67) | 32,01 |
| | 12,50 | 3/11 ( 27) | |
| | 6,25 | 0/11 ( 0) | |

**In vivo-Wirksamkeit von Mersacidin gegen S. aureus SG 511 Abszeß in Ratten im Vergleich zu Vancomycin.**

**Verwendetes Impfmedium: 2 x $10^9$ KBU in 5 % Mucin unter die Haut injiziert.**

Behandlung: 50 mg/kg x 7 Tage
Ergebnis: Mersacidin vermindert die Bakterienbildung um 1.4 $\log_{10}$ KBU/ml (Kolonie bildende Einheiten), Vancomycin reduziert die Bakterienbildung um 0,79 $\log_{10}$ KBU/ml.

**BEISPIEL 6**

Herstellung von wasserlöslichen Salzen des Mersacidins

Mersacidin ist wasserlöslich in Form seiner Alkali-Salze. Diese lassen sich herstellen z.B. durch die Umsetzung des Mersacidins mit einem Äquivalent des entsprechenden Alkalimetallhydroxyds in Gegenwart von Pyridin. Z.B. wurden 50 mg Mersacidin in 1ml Pyridin gelöst, die Lösung wurde auf 0 °C gekühlt und eine Lösung von 1,683 mg KOH in 3 ml destilliertem Wasser wurde unter Rühren hinzugefügt. Die Mischung wurde bei 0°C für 1 Stunde gerührt und anschließend lyophilisiert. Es wurden 49 mg Kalium-Mersacidin in Form eines weißen Pulvers erhalten.
Dieses Kalium-Mersacidin ist löslich bis 160 mg/ml Wasser. Die in vitro und in vivo antibakteriellen Eigenschaften des Kalium-Mersacidins sind vergleichbar mit denen des Mersacidins. Versuchsergebnisse zeigen die Tabellen 5 und 6.

Tabelle 5

| MHK-Werte von Kalium-Mersacidin | | |
|---|---|---|
| Nr. | Testorganismus | MHK-Werte (µg/ml) |
| 1. | Staph. aureus 209P | 2 |
| 2. | Staph. aureus R 85 | 2 |
| 3. | Staph. aureus 710 | 1 |
| 4. | Staph. aureus 3066 | 10 |
| 5. | Staph. aureus 25923 | 8 |
| 6. | Staph. epidermidis 32965 | 6 |
| 7. | Staph. epidermidis 823 | 50 |
| 8. | Staph. haemolyticus 712 | 10 |
| 9. | Staph. haemolyticus 809 | 4 |
| 10. | Strepto. hominis ML 22 | 10 |
| 11. | Strepto. faecalis 29212 | 15 |
| 12. | Strepto. faecalis 21777 | 15 |

Tabelle 6

| In vivo Wirksamkeit von Kalium-Mersacidin gegen S. aureus und Strept. pyogenes A 77 Infektionen in Mäusen | | |
|---|---|---|
| Testorganismus | Verwendetes Impfmedium | 50 % Effectiv Dosis ($ED_{50}$) mg/kgx 3, s.c. |
| Staph. aureus SG 511 (Methicillin sensitive) | 1,75 x $10^9$ | 2,85 |
| Staph. aureus E 710 (Methicillin resistant) | 1 x $10^{10}$ | 6,72 |
| Staph. aureus C 31153 (Methicillin & Caphalexin resistent) | 2 x $10^9$ | 15,35 |
| Strept. pyogenes A 77 | 3,8 x $10^3$ | 0,46 |

**Patentansprüche**

**Patentansprüche für folgende Vertragsstaaten : AT, BE, CH, DE, FR, GB, IT, LI, LU, NL, SE**

1. Eine Verbindung der Formel I

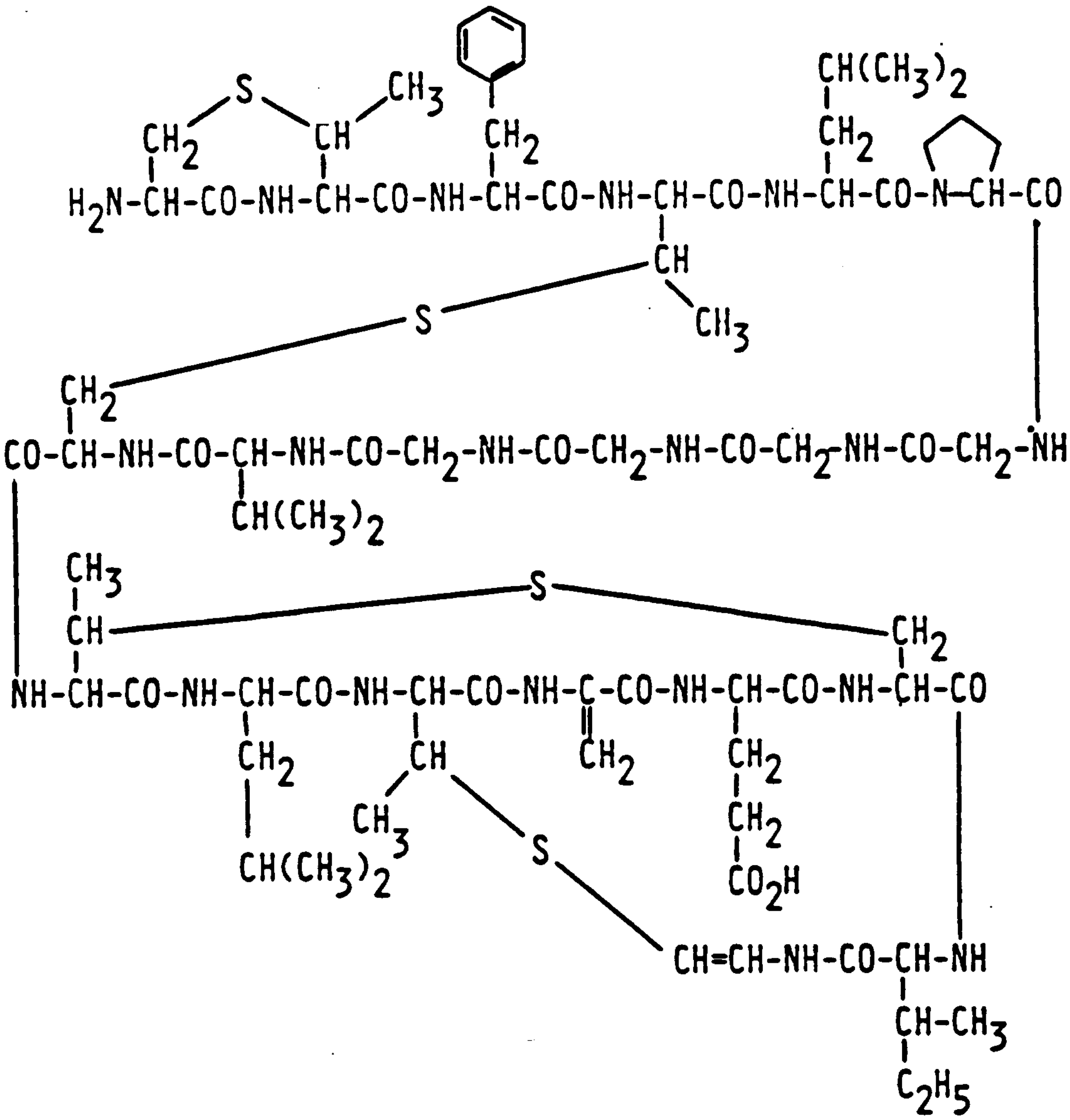

sowie deren physiologisch verträgliche Salze.

**2.** Eine Verbindung der Formel II

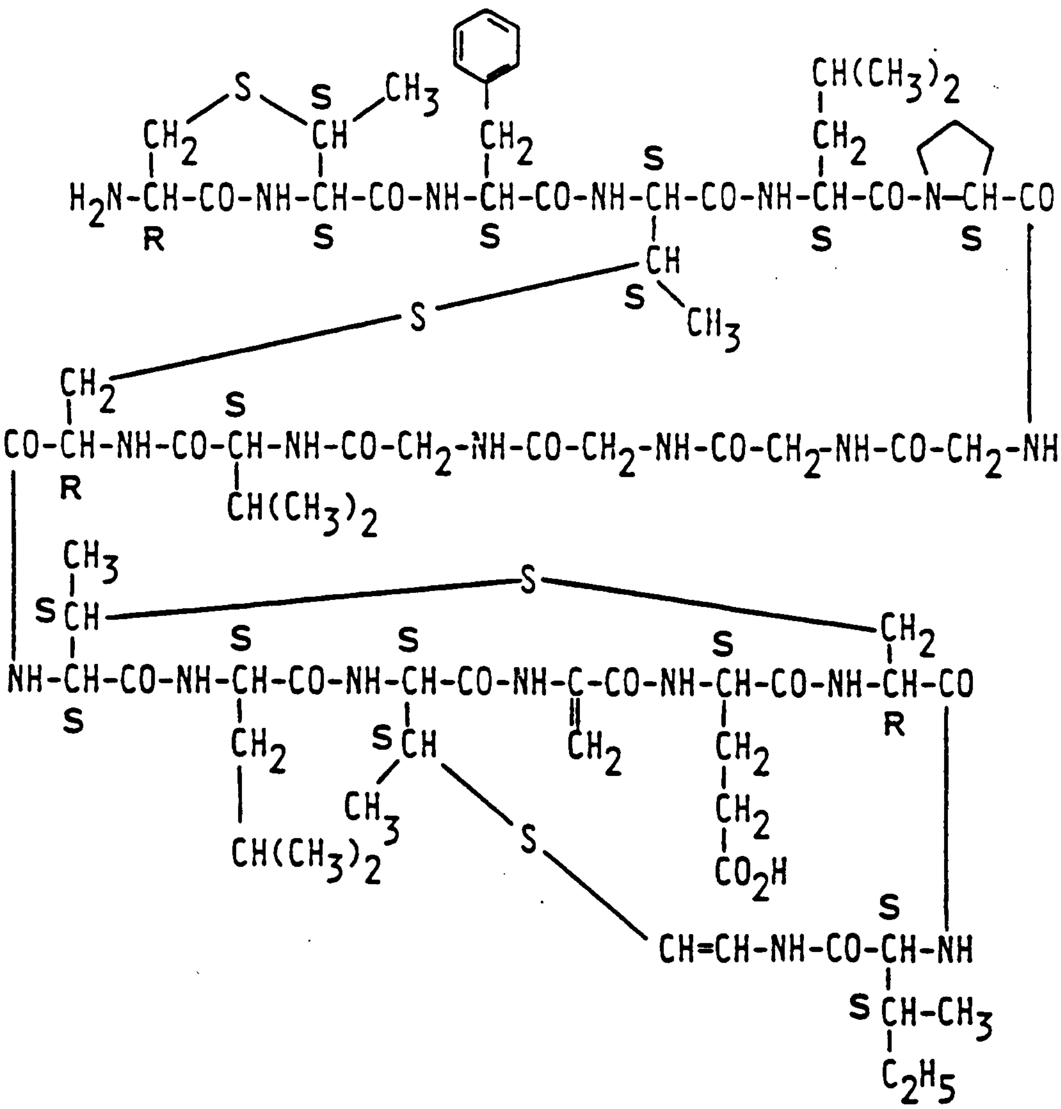

(II)

sowie deren physiologisch verträgliche Salze.

**3.** Verfahren zur Herstellung der Verbindung der Formel I oder II gemäß den Ansprüchen 1 oder 2, dadurch gekennzeichnet, daß man das Eubacterium Bacillus sp. Y-85,54728 (DSM 4584), deren Mutanten und/oder Varianten unter aeroben Bedingungen in einem Kohlenstoff- und Stickstoffquellen, anorganische Nährsalze und Spurenelemente enthaltenden Nährmedium kultiviert und die Verbindung in üblicher Weise aus der Kulturbrühe isoliert und reinigt.

**4.** Verfahren nach Anspruch 3, dadurch gekennzeichnet, daß die Kultivierung bei Temperaturen zwischen ca. 26 und 29 °C und bei einem pH-Wert zwischen ca. 6,5 und 7,2 durchgeführt wird.

**5.** Verfahren nach den Ansprüchen 3 oder 4, dadurch gekennzeichnet, daß die Kultivierung bei 28 °C (±1 °C) und pH ca. 7,2 durchgeführt wird.

**6.** Verfahren nach einem oder mehreren der Ansprüche 3-5, dadurch gekennzeichnet, daß die Fermentation länger als 60 Stunden durchgeführt wird.

**7.** Verfahren nach einem oder mehreren der Ansprüche 3-6, dadurch gekennzeichnet, daß die Verbindungen der Formel I und II durch chromatographische Methoden gereinigt werden.

8. Arzneimittel, gekennzeichnet durch einen Gehalt an einer Verbindung gemäß Anspruch 1 oder Anspruch 2, gegebenenfalls neben üblichen Hilfs- und/oder Trägerstoffen für die Arzneimittelherstellung.

9. Verwendung der Verbindung gemäß Anspruch 1 oder 2 zur Herstellung von Arzneimitteln mit antibiotischer Wirkung.

10. Bacillus species Y-85,54728 (DSM 4584).

**Patentansprüche für folgende Vertragsstaaten: ES, GR**

1. Verfahren zur Herstellung einer Verbindung der Formel I

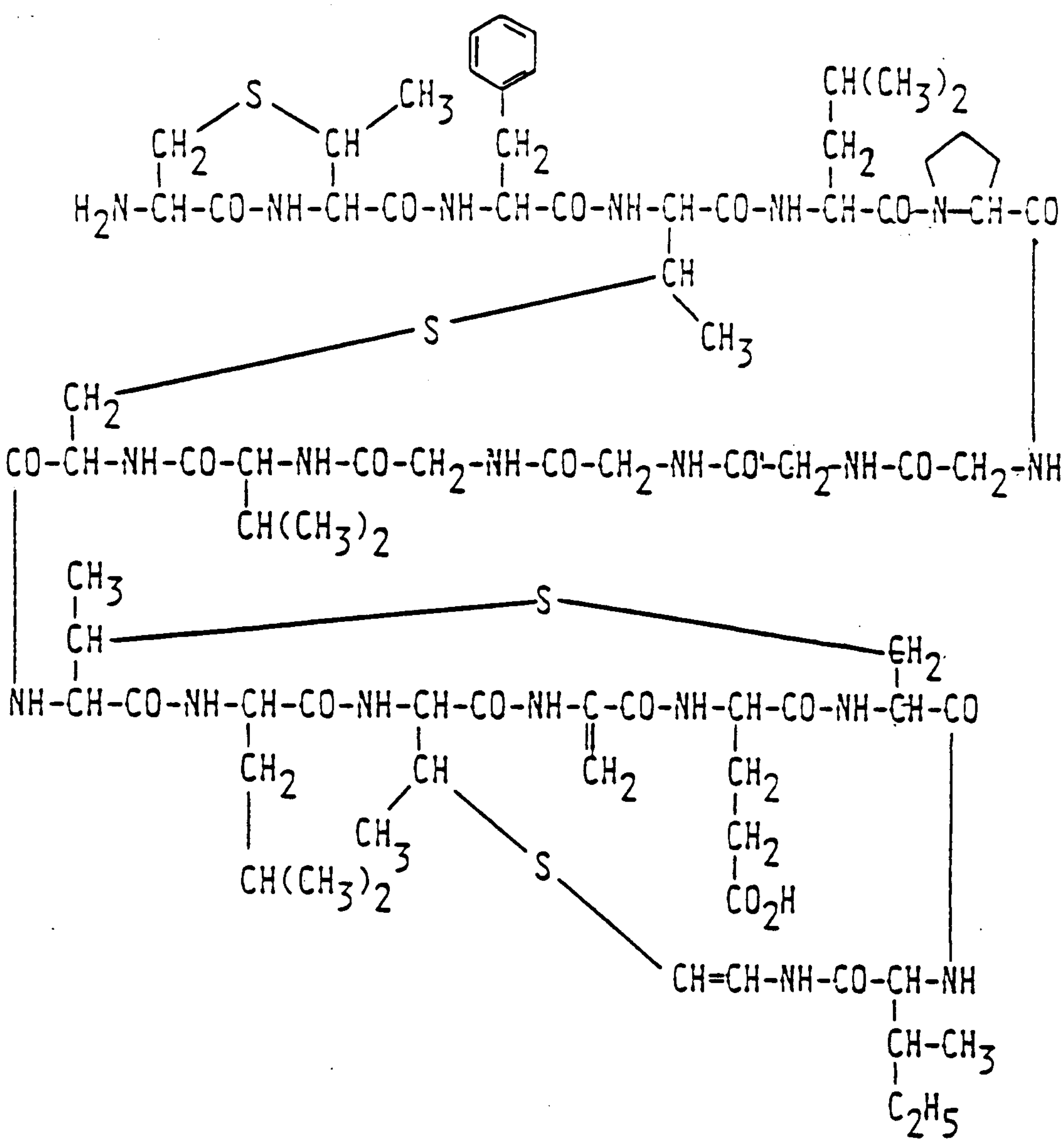

dadurch gekennzeichnet, daß man das Eubacerium Bacillus sp. Y-85,54728 (DSM 4584), deren Mutanten und/oder Varianten unter aeroben Bedingungen in einem Kohlenstoff- und Stickstoffquellen, anorganische Nährsalze und Spurenelemente enthaltenden Nährmedium kultiviert und die Verbindung in üblicher Weise aus der Kulturbrühe isoliert und reinigt.

**2.** Verfahren zur Herstellung einer Verbindung der Formel II

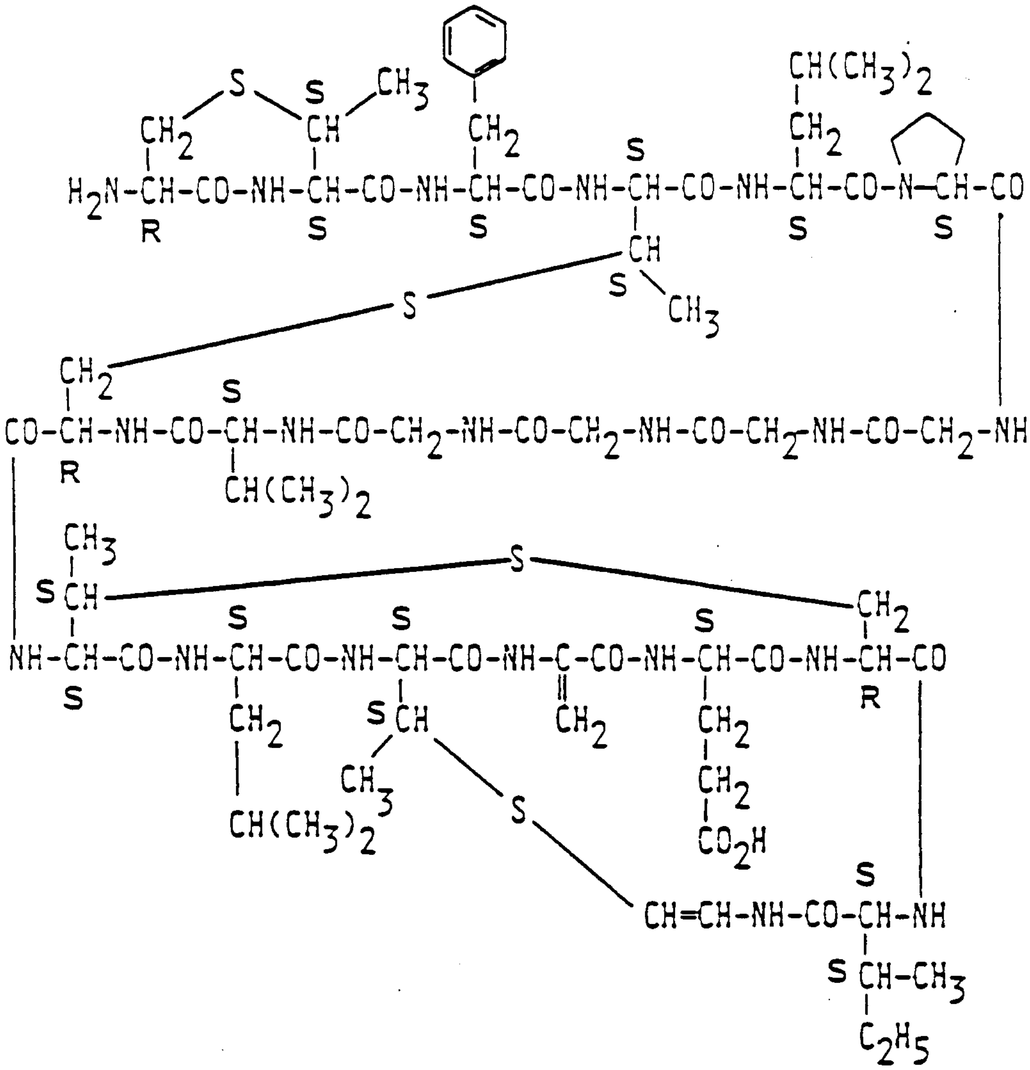

(II)

dadurch gekennzeichnet, daß man das Eubacerium Bacillus sp. Y-85,54728 (DSM 4584), deren Mutanten und/oder Varianten unter aeroben Bedingungen in einem Kohlenstoff- und Stickstoffquellen, anorganische Nährsalze und Spurenelemente enthaltenden Nährmedium kultiviert und die Verbindung in üblicher Weise aus der Kulturbrühe isoliert und reinigt.

**3.** Verfahren nach Anspruch 1 oder 2, dadurch gekennzeichnet, daß die Kultivierung bei Temperaturen zwischen ca. 26 und 29 °C und bei einem pH-Wert zwischen ca. 6,5 und 7,2 durchgeführt wird.

**4.** Verfahren nach einem oder mehreren der Ansprüche 1 bis 3, dadurch gekennzeichnet, daß die Kultivierung bei 28 °C (±1 °C) und pH ca. 7,2 durchgeführt wird.

**5.** Verfahren nach einem oder mehreren der Ansprüche 1-4, dadurch gekennzeichnet, daß die Fermentation länger als 60 Stunden durchgeführt wird.

**6.** Verfahren nach einem oder mehreren der Ansprüche 1-5, dadurch gekennzeichnet, daß die Verbindungen der Formel I und II durch chromatographische Methoden gereinigt werden.

**7.** Verfahren zur Herstellung von Arzneimitteln, dadurch gekennzeichnet, daß man eine Verbindung hergestellt gemäß einem oder mehreren der Ansprüche 1-6, gegebenenfalls mit üblichen Hilfs- und/oder Trägerstoffen für die Arzneimittelherstellung in eine geeignete Darreichungsform bringt.

8. Verwendung einer Verbindung hergestellt gemäß einem oder mehreren der Ansprüche 1 bis 6 zur Herstellung von Arzneimitteln mit antibiotischer Wirkung.

9. Bacillus species Y-85,54728 (DSM 4584).

## Claims

**Claims for the following Contracting States : AT, BE, CH, DE, FR, GB, IT, LI, LU, NL, SE**

1. A compound of the formula I

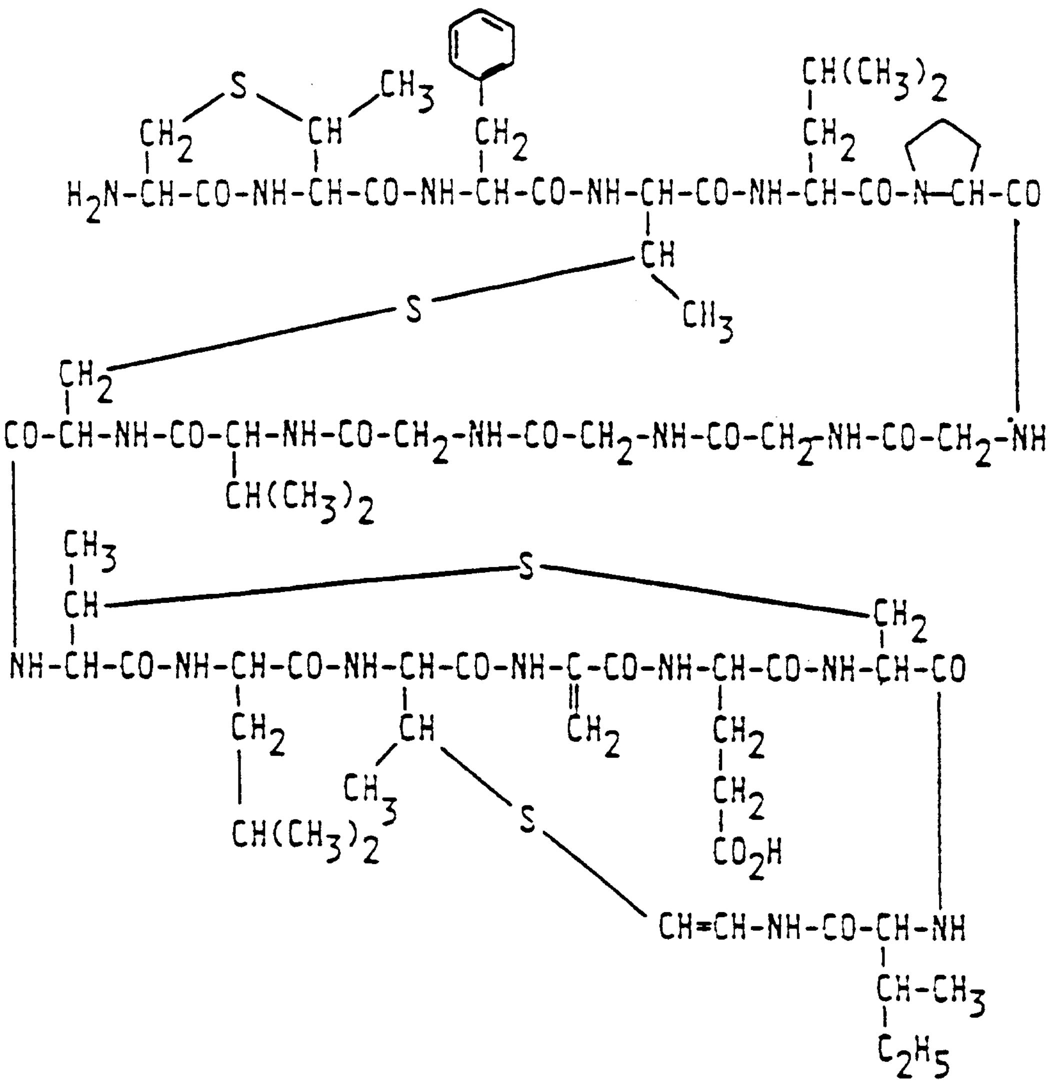

as well as the physiologically tolerated salts thereof.

**2.** A compound of the formula II

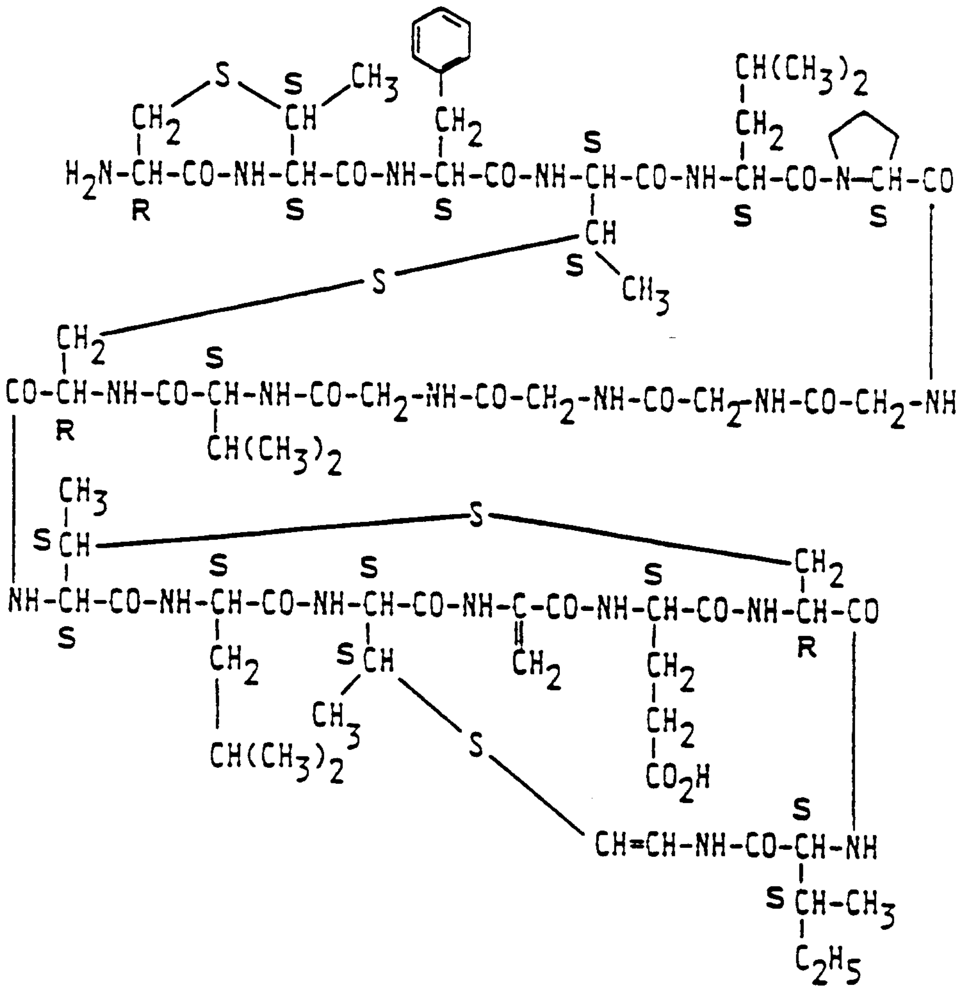

(II)

as well as the physiologically tolerated salts thereof.

**3.** A process for the preparation of the compound of the formula I or II as claimed in claims 1 or 2, which comprises the Eubacterium Bacillus sp. Y-85,54728 (DSM 4584), the mutants and/or variants thereof being cultivated under aerobic conditions in a nutrient medium containing sources of carbon and of nitrogen, inorganic nutrient salts and trace elements, and the compound being isolated from the culture broth and purified in a customary manner.

**4.** The process as claimed in claim 3, wherein the cultivation is carried out at temperatures between about 26 and 29 °C and at a pH between about 6.5 and 7.2.

**5.** The process as claimed in claims 3 or 4, wherein the cultivation is carried out at 28 °C (± 1 °C) and pH about 7.2.

**6.** The process as claimed in one or more of claims 3-5, wherein the fermentation is carried out for more than 60 hours.

**7.** The process as claimed in one or more of claims 3-6, wherein the compounds of the formula I and II are purified by chromatographic methods.

**8.** A pharmaceutical which contains a compound as claimed in claim 1 or claim 2, where appropriate in addition to auxiliaries and/or excipients customary for the preparation of pharmaceuticals.

9. The use of the compound as claimed in claim 1 or 2 for the preparation of pharmaceuticals having an antibiotic action.

10. Bacillus species Y-85,54728 (DSM 4584).

**Claims for the following Contracting States : ES, GR**

1. A process for the preparation of a compound of the formula I

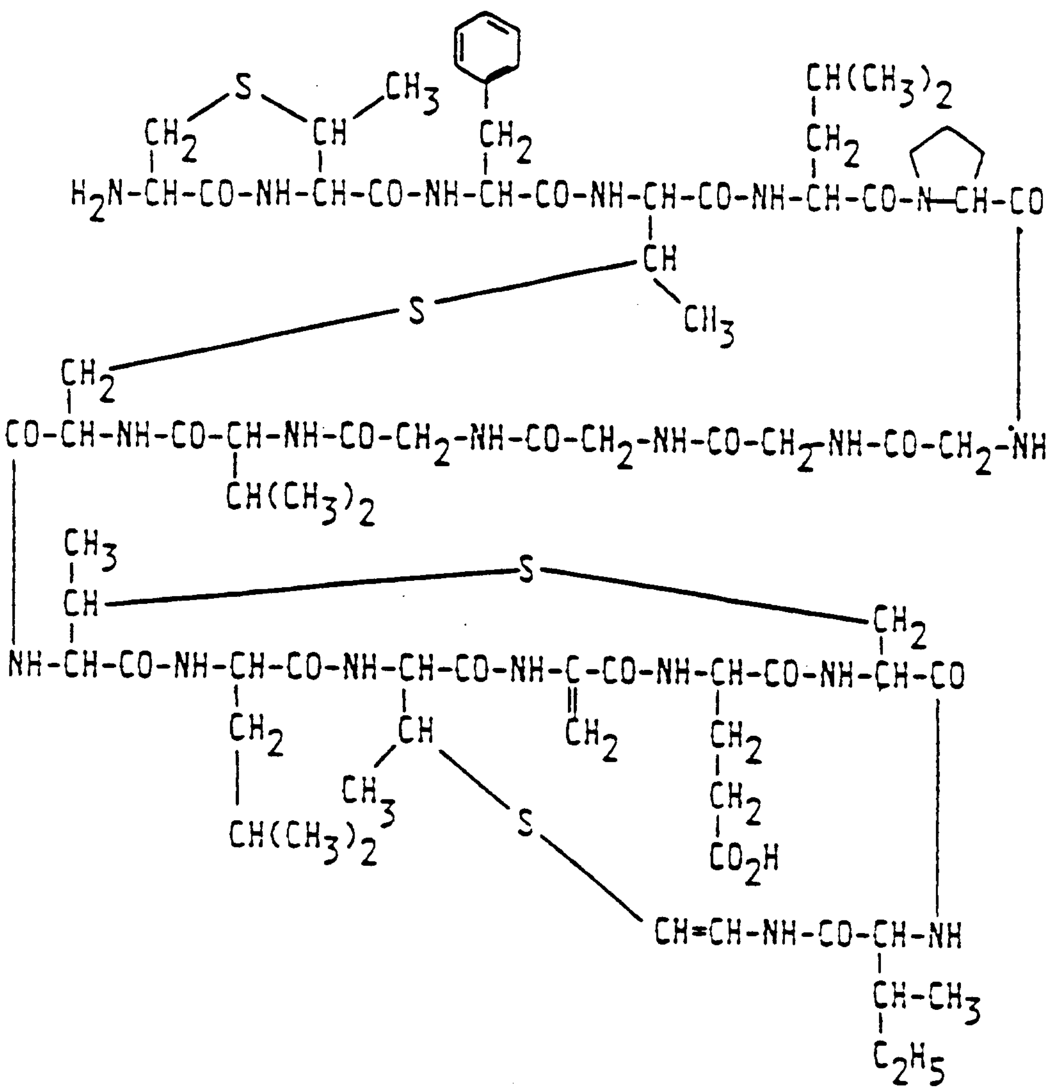

which comprises the Eubacterium Bacillus sp. Y-85,54728 (DSM 4584), the mutants and/or variants thereof being cultivated under aerobic conditions in a nutrient medium containing sources of carbon and of nitrogen, inorganic nutrient salts and trace elements, and the compound being isolated from the culture broth and purified in a customary manner.

2. A process for the preparation of a compound of the formula II

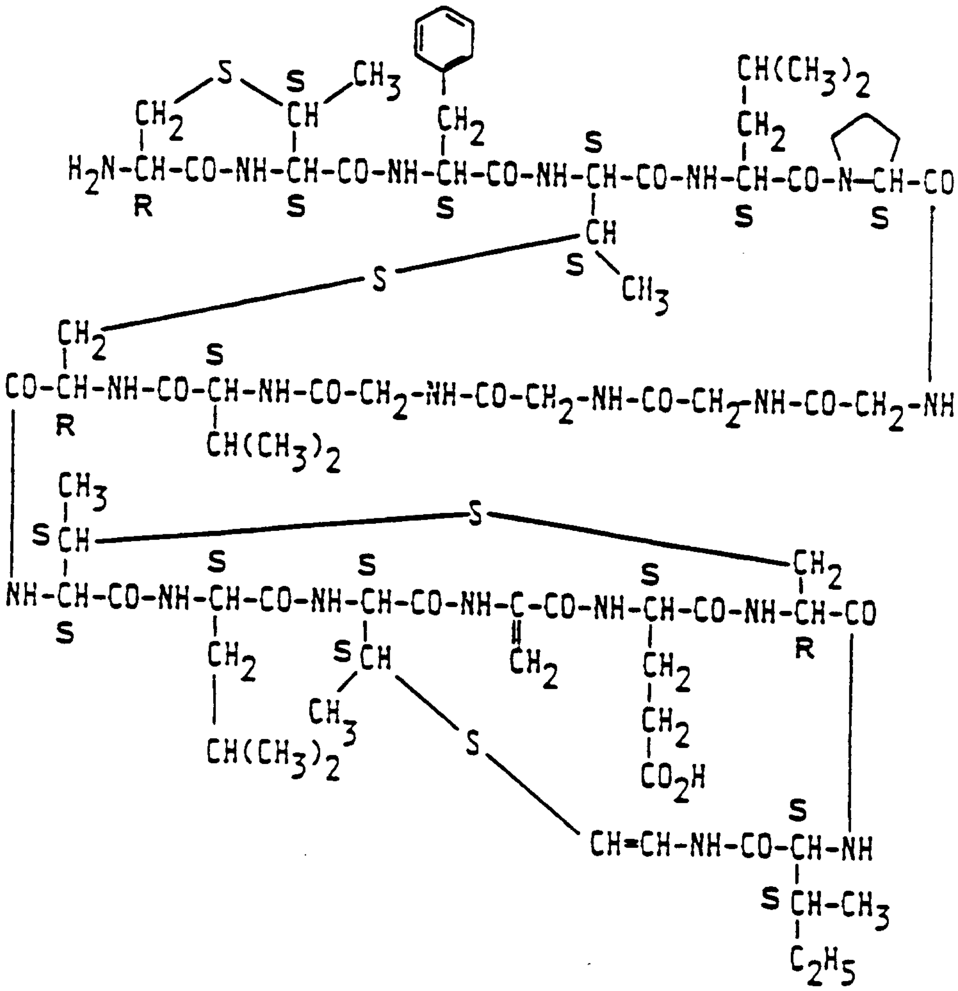

(II)

which comprises the Eubacterium Bacillus sp. Y-85,54728 (DSM 4584), the mutants and/or variants thereof being cultivated under aerobic conditions in a nutrient medium containing sources of carbon and of nitrogen, inorganic nutrient salts and trace elements, and the compound being isolated from the culture broth and purified in a customary manner.

3. The process as claimed in claim 1 or 2, wherein the cultivation is carried out at temperatures between about 26 and 29 °C and at a pH between about 6.5 and 7.2.

4. The process as claimed in one or more of claims 1 to 3, wherein the cultivation is carried out at 28 °C (± 1 °C) and pH about 7.2.

5. The process as claimed in one or more of claims 1-4, wherein the fermentation is carried out for more than 60 hours.

6. The process as claimed in one or more of claims 1-5, wherein the compounds of the formula I and II are purified by chromatographic methods.

7. A process for the preparation of a pharmaceutical, which comprises a compound prepared as claimed in one or more of claims 1-6 being converted, where appropriate with auxiliaries and/or excipients customary for the preparation of pharmaceuticals, into a suitable administration form.

8. The use of a compound prepared as claimed in one or more of claims 1 to 6 for the preparation of pharmaceuticals having an antibiotic action.

**9.** Bacillus species Y-85,54728 (DSM 4584).

**Revendications**

**Revendications pour les Etats contractants suivants : AT, BE, CH, DE, FR, GB, IT, LI, LU, NL, SE**

**1.** Composé de formule I

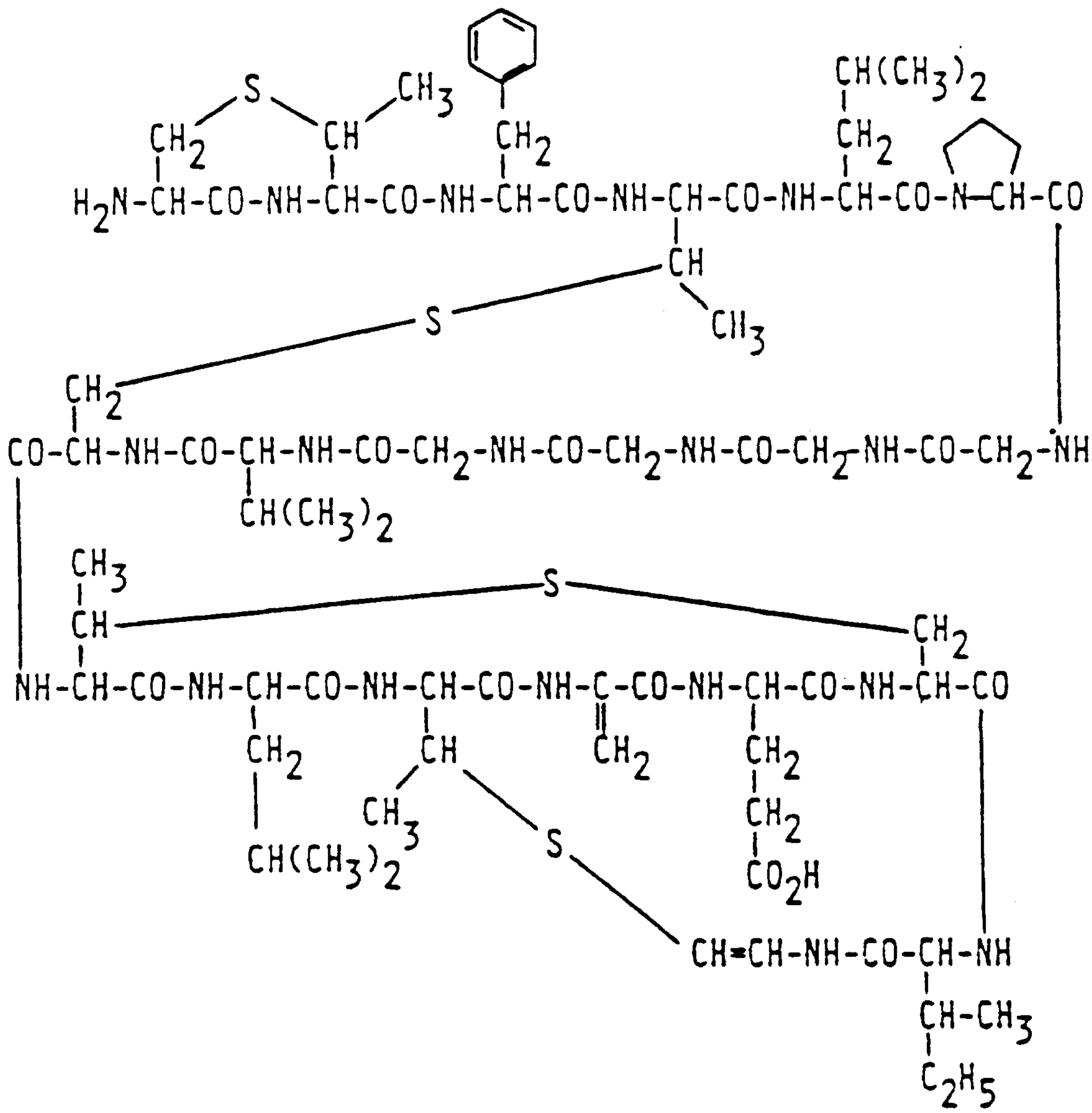

et sels physiologiquement acceptables de celui-ci.

2. Composé de formule II

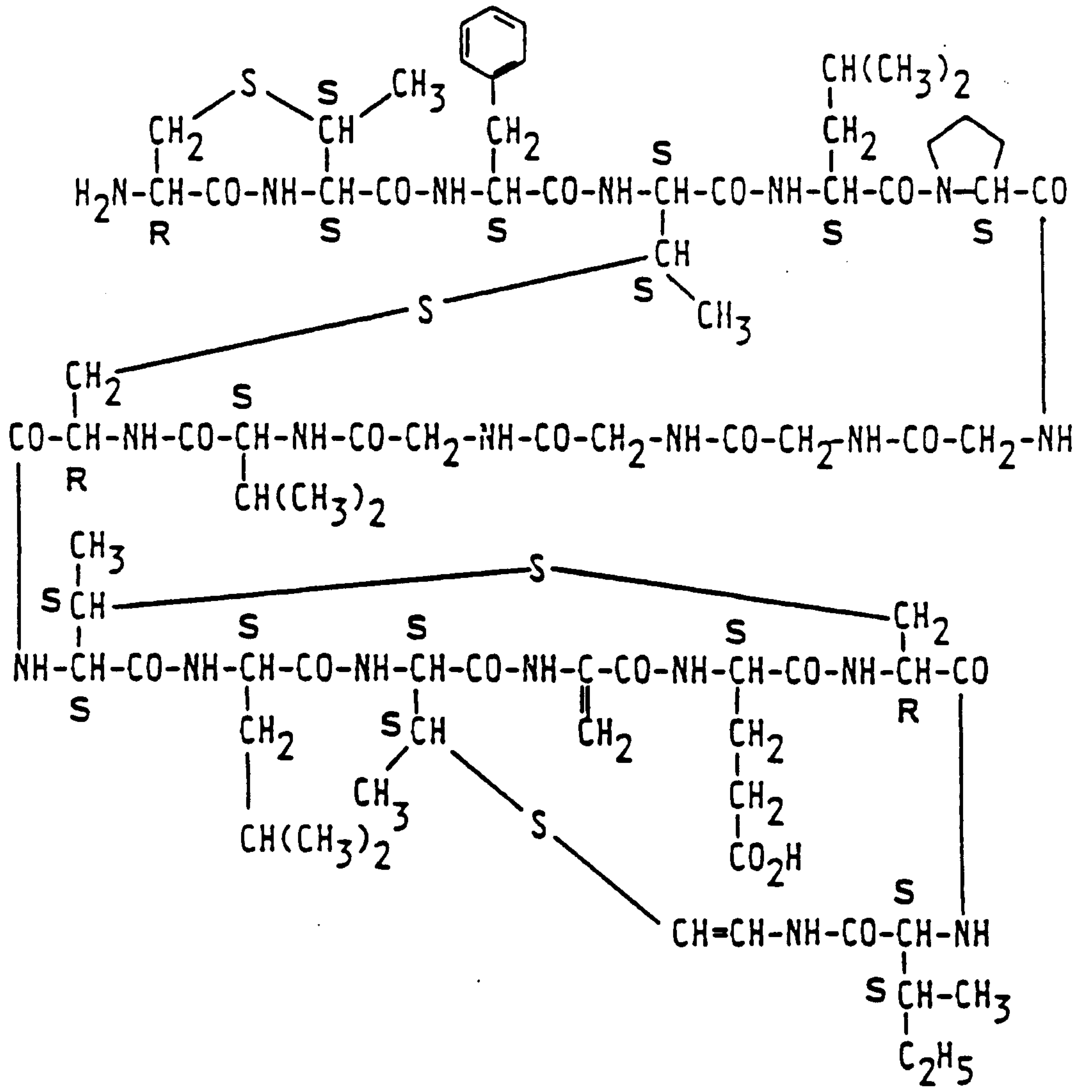

(II)

et sels physiologiquement acceptables de celui-ci.

3. Procédé pour la production du composé de formule I ou II selon la revendication 1 ou 2, caractérisé en ce que l'on cultive dans des conditions aérobies l'eubactérie Bacillus sp. Y-85,54728 (DSM 4584), ses mutants et/ou variants, dans un milieu nutritif contenant des sources de carbone et d'azote, des sels minéraux nutritifs et des oligo-éléments, et on isole à partir du bouillon de culture et purifie le composé à la manière usuelle.

4. Procédé selon la revendication 3, caractérisé en ce que la culture est effectuée à des températures comprises entre environ 26 et 29 °C et à un pH compris entre environ 6,5 et 7,2.

5. Procédé selon la revendication 3 ou 4, caractérisé en ce que la culture est effectuée à 28 °C (±1 °C) et à un pH d'environ 7,2.

6. Procédé selon une ou plusieurs des revendications 3 à 5, caractérisé en ce que la fermentation est effectuée pendant plus de 60 heures.

7. Procédé selon une ou plusieurs des revendications 3 à 6, caractérisé en ce que les composés de formules I et II sont purifiés par des méthodes chromatographiques.

8. Médicament, caractérisé par une teneur en un composé selon la revendication 1 ou la revendication 2, éventuellement en plus de véhicules et/ou adjuvants usuels pour la fabrication de médicaments.

9. Utilisation du composé selon la revendication 1 ou 2, pour la fabrication de médicaments à activité antibiotique.

10. Bacillus sp. Y-85,54728 (DSM 4584).

**Revendications pour les Etats contractants suivants : ES, GR**

1. Procédé pour la production d'un composé de formule I

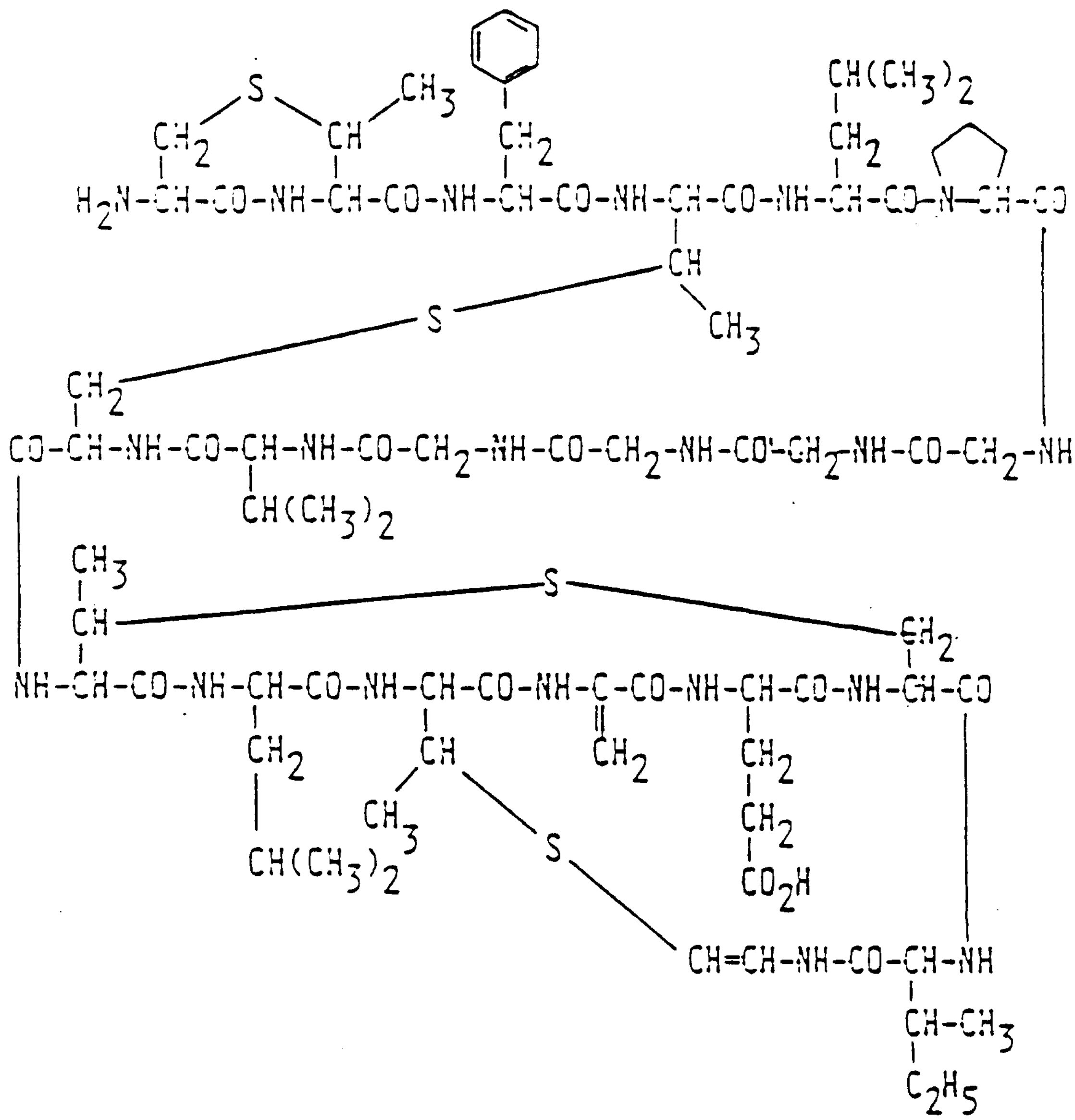

caractérisé en ce que l'on cultive dans des conditions aérobies l'eubactérie Bacillus sp. Y-85,54728 (DSM 4584), ses mutants et/ou variants, dans un milieu nutritif contenant des sources de carbone et d'azote, des sels minéraux nutritifs et des oligoéléments, et on isole à partir du bouillon de culture et purifie le composé à la manière usuelle.

2. Procédé pour la production d'un composé de formule II

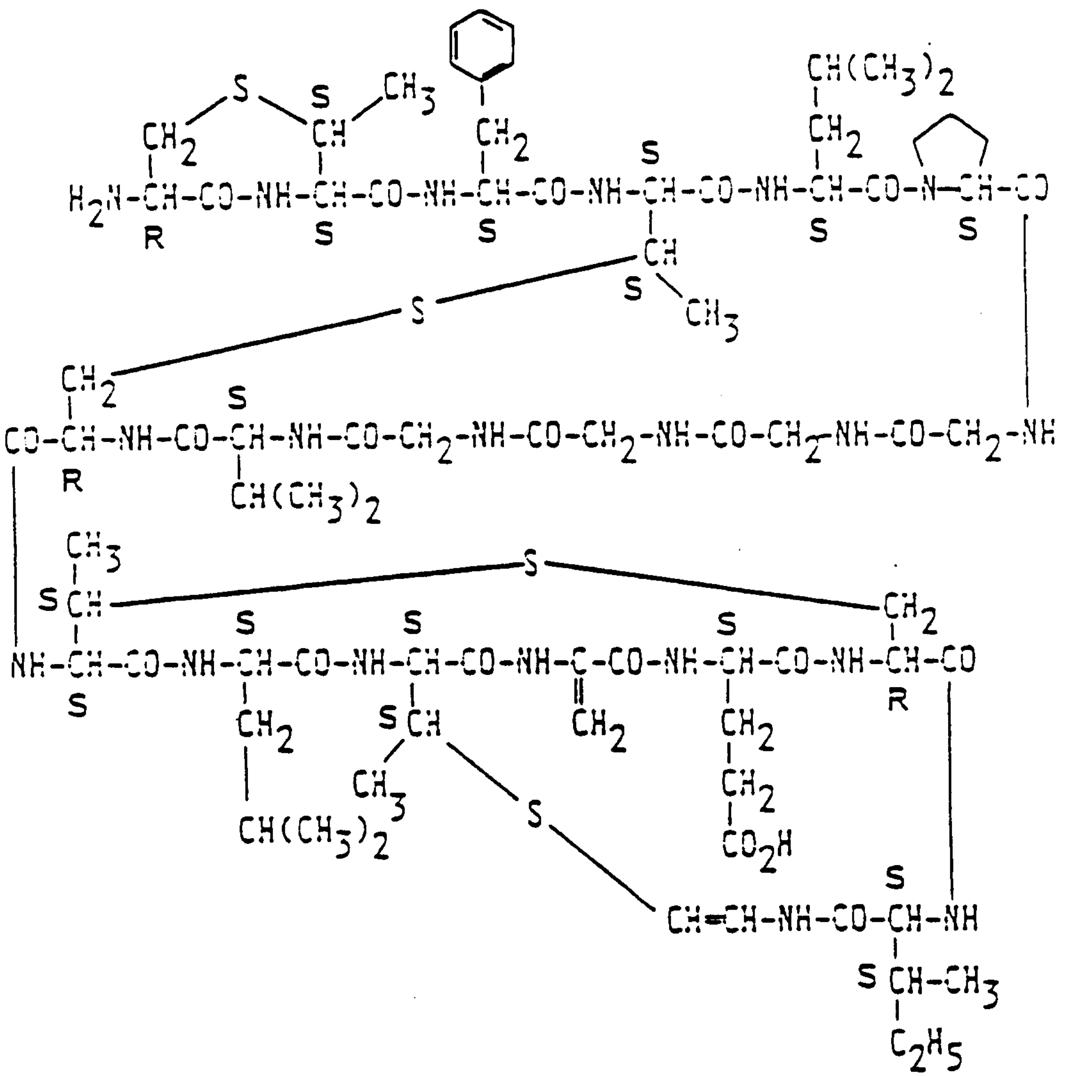

(II)

caractérisé en ce que l'on cultive dans des conditions aérobies l'eubactérie Bacillus sp. Y-85,54728 (DSM 4584), ses mutants et/ou variants, dans un milieu nutritif contenant des sources de carbone et d'azote, des sels minéraux nutritifs et des oligoéléments, et on isole à partir du bouillon de culture et purifie le composé à la manière usuelle.

3. Procédé selon la revendication 1 ou 2, caractérisé en ce que la culture est effectuée à des températures comprises entre environ 26 et 29 ° C et à un pH compris entre environ 6,5 et 7,2.

4. Procédé selon une ou plusieurs des revendications 1 à 3, caractérisé en ce que la culture est effectuée à 28 ° C (±1 ° C) et à un pH d'environ 7,2.

5. Procédé selon une ou plusieurs des revendications 1 à 4, caractérise en ce que la fermentation est effectuée pendant plus de 60 heures.

6. Procédé selon une ou plusieurs des revendications 1 à 5, caractérisé en ce que les composés de formules I et II sont purifiés par des méthodes chromatographiques.

7. Procédé pour la fabrication d'un médicament, caractérisé en ce que l'on met sous une forme d'administration appropriée un composé produit selon une ou plusieurs des revendications 1 à 6, éventuellement avec des véhicules et/ou adjuvants usuels pour la fabrication de médicaments.

8. Utilisation d'un composé produit selon une ou plusieurs des revendications 1 à 6, pour la fabrication de médicaments à activité antibiotique.

9. Bacillus sp. Y-85,54728 (DSM 4584).

Analytische HPLC-Kurve von Mersacidin auf einer APS.-Hypersil (5 µ) Säule, Elutionsmittel Methanol: Wasser (70:30), Fluß-Rate 1 ml / Min; Detektion bei 210 nm, Schreibergeschwindigkeit 10 mm / Min.

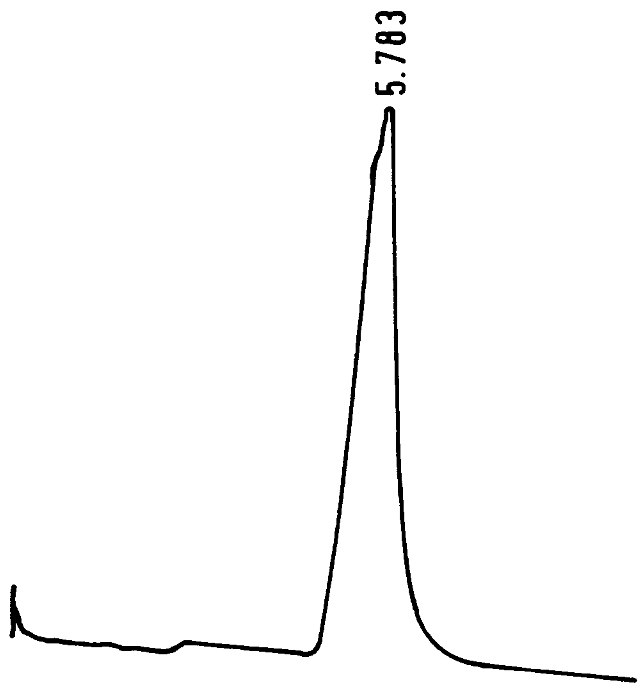


Fig. 1

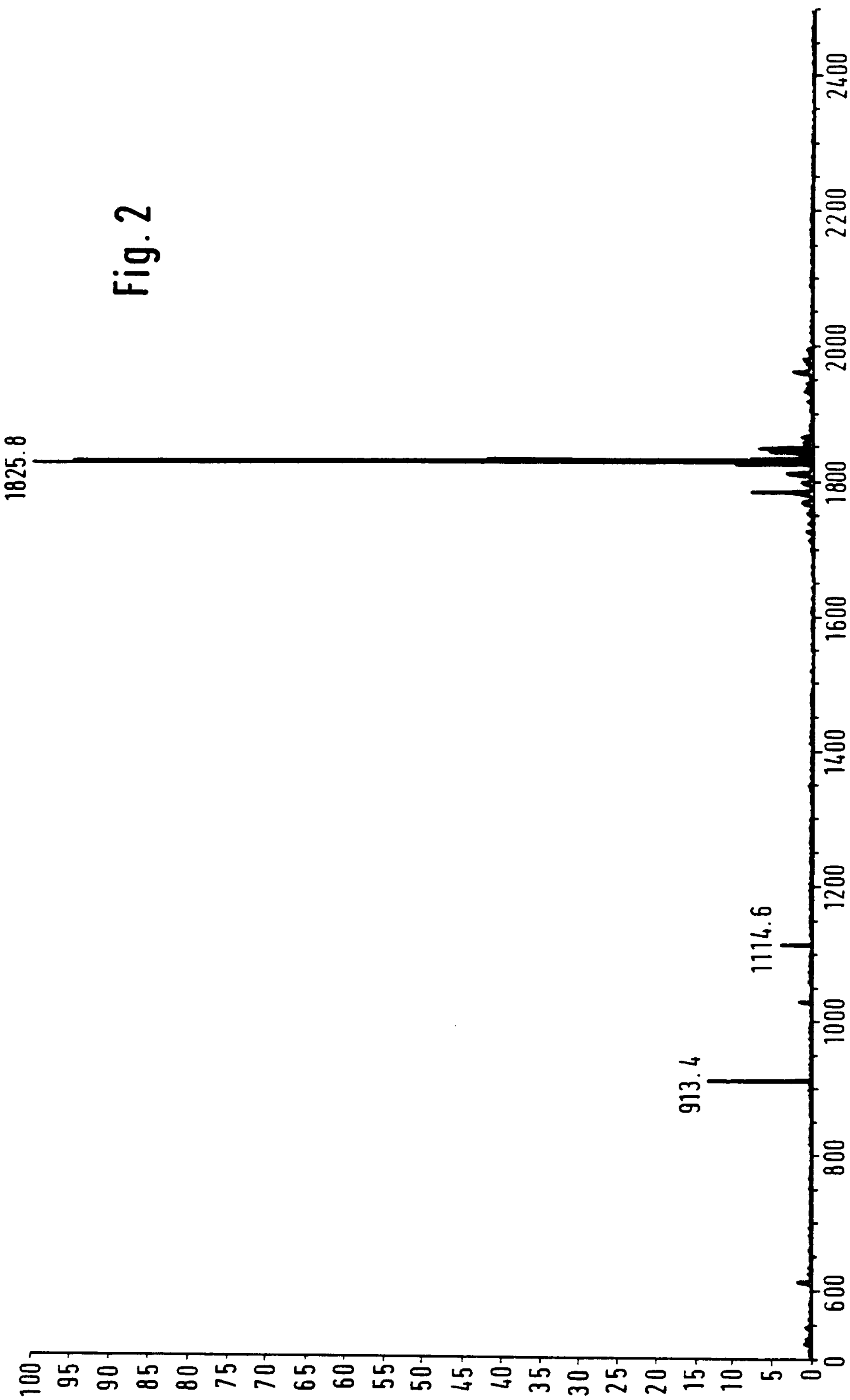
1825.8
1114.6
913.4
100
95
90
85
80
75
70
65
60
55
50
45
40
35
30
25
20
15
10
5
0
0
600
800
1000
1200
1400
1600
1800
2000
2200
2400

Fig. 2

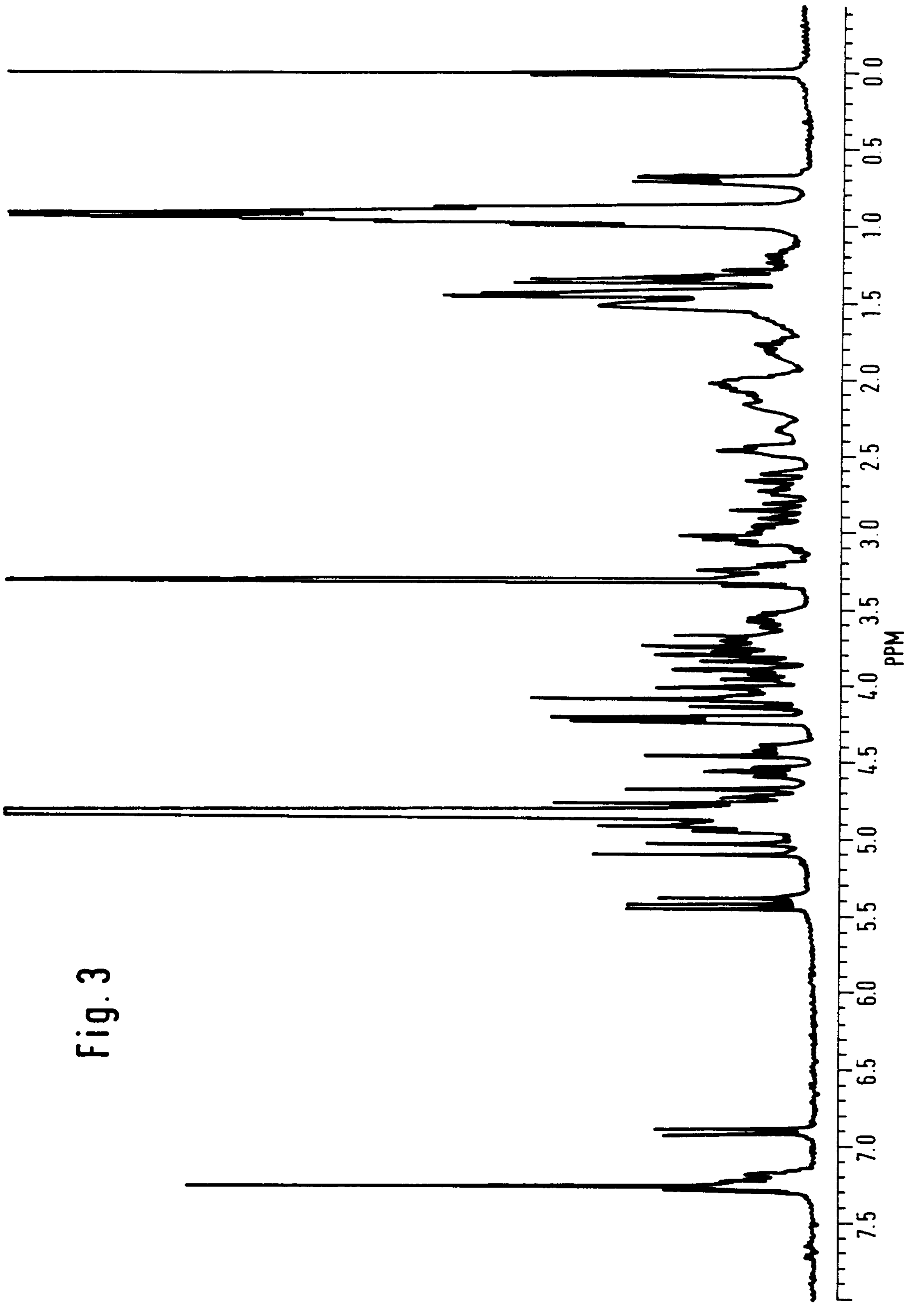


Fig. 3